# EUROPEAN PATENT SPECIFICATION

(11) **EP 1 700 598 B1**
(45) Date of publication and mention of the grant of the patent: **13.05.2009**
(21) Application number: 05005380.0
(22) Date of filing: 11.03.2005
(51) Int. Cl.: A61K 31/282, A61K 31/337, A61P 35/00

(54) **Anti-proliferative combination therapy comprising satraplatin or JM118 and docetaxel**
Antiproliferative Kombinations-Therapie mit Satraplatin oder JM118 und Docetaxel
Therapie anti-proliférative combinée avec le satraplatine ou JM118 et le docetaxel

(43) Date of publication of application: 13.09.2006
(73) Proprietor: GPC Biotech AG, 82152 Martinsried (DE)
(72) Inventor: Wosikowski-Buters, Katja, 85586 Poing (DE); Shah, Hemanshu, Princeton, NJ 08540 (US); Caligiuri, Maureen, Reading, MA 01867 (US)
(74) Representative: Dörries, Hans Ulrich

(56) References cited:
- JONES SUZANNE ET AL: "Phase I study of JM-216 (an oral platinum analogue) in combination with paclitaxel in patients with advanced malignancies" INVESTIGATIONAL NEW DRUGS, vol. 20, no. 1, February 2002 (2002-02), pages 55-61, XP002336031 ISSN: 0167-6997
- ENGBLOM P ET AL: "Additive and supra-additive cytotoxicity of cisplatin-taxane combinations in ovarian carcinoma cell lines." BRITISH JOURNAL OF CANCER. JAN 1999, vol. 79, no. 2, January 1999 (1999-01), pages 286-292, XP002336032 ISSN: 0007-0920
- ENGBLOM P ET AL: "CARBOPLATIN-PACLITAXEL- AND CABOPLATIN-DOCETAXEL-INDUCED CYTOTOXIC EFFECT IN EPITHELIAL OVARIAN CARCINOMA IN VITRO" CANCER, AMERICAN CANCER SOCIETY, PHILADELPHIA, PA, US, vol. 86, no. 10, 1999, pages 2066-2073, XP001021402 ISSN: 0008-543X
- VAN PUTTEN J W G ET AL: "Docetaxel and carboplatin combination as second-line treatment in metastatic non-small-cell lung cancer(NSCLC)" EUROPEAN JOURNAL OF CANCER, PERGAMON PRESS, OXFORD, GB, vol. 37, April 2001 (2001-04), page S51, XP004477419 ISSN: 0959-8049
- MORENO J A ET AL: "Phase II study of docetaxel and cisplatin in first line treatment of disseminated small cell lung cancer (SCLC): preliminary results" EUROPEAN JOURNAL OF CANCER, PERGAMON PRESS, OXFORD, GB, vol. 35, September 1999 (1999-09), page S262, XP004384955 ISSN: 0959-8049
- LATIF TAHIR ET AL: "Phase II study of oral bis (aceto) ammine dichloro (cyclohexamine) platinum (IV) (JM-216, BMS-182751) given daily x 5 in hormone refractory prostate cancer (HRPC)" INVESTIGATIONAL NEW DRUGS, vol. 23, no. 1, January 2005 (2005-01), pages 79-84, XP002336033 ISSN: 0167-6997
- KELLAND L R: "AN UPDATE ON SATRAPLATIN: THE FIRST ORALLY AVAILABLE PLATINUM ANTICANCER DRUG" EXPERT OPINION ON INVESTIGATIONAL DRUGS, ASHLEY PUBLICATIONS LTD., LONDON, GB, vol. 9, no. 6, June 2000 (2000-06), pages 1373-1382, XP001097881 ISSN: 1354-3784

## Description

This invention relates to a method of prevention and/or treatment of a cancer or a tumor, and in particular to a combination therapy, methods, compositions and pharmaceutical packages comprising a taxane and certain platinum-based chemotherapeutic agents.

### Background of the invention

Platinum compounds are among the most active chemotherapeutic agents available for the treatment of a variety of cancers and tumors. The use of some of these compounds, e.g., cisplatin, is restricted by both toxological and resistance considerations. To overcome these issues, efforts were started to discover novel platinum compounds which do not share certain properties of cisplatin. One compound that was identified is satraplatin (JM216), a platinum (Pt) IV complex. Satraplatin (JM216) was selected for clinical development because of several advantageous properties: (a) high cytotoxic activity in vitro against several solid tumor cell lines, including cisplatin-resistant ones; (b) in vivo oral antitumor activity against a variety of murine- and human-xenograft tumor models; (c) a relatively mild toxicity profile (such as the absence of kidney toxicity and neurotoxicity), and (d) oral availability.

In Phase 2 clinical trials, satraplatin showed activity against several different cancers, including prostate, ovarian, and small cell lung (SCL) cancers. In a Phase II-III clinical trial in Hormone Refractory Prostate Carcinoma (HRPC) patients, the combination of satraplatin plus prednisone was more active than prednisone alone (ASCO meeting, 2003; Stemberg et al., Onclogy (2005) 68, 2). Satraplatin is currently undergoing Phase 3 development in a worldwide registration clinical study evaluating satraplatin plus prednisone versus placebo plus prednisone as second-line cytotoxic chemotherapeutic treatment against hormone refractory prostate cancer. The current standard treatment of HRPC is primarily palliative and includes first line chemotherapeutic regimens with agents such as estramustine, mitoxantrone and taxanes, with docetaxel being increasingly used as a first-line chemotherapeutic agent.

Satraplatin is considerably different from other platinum agents, like e.g. cisplatin. Using a panel of ovarian cancer carcinoma cell lines Kelland et al. (Cancer Res (1992), 52, 822) demonstrated that satraplatin is significantly more cytotoxic than cisplatin, and that satraplatin exhibits selective cytotoxic effects against intrinsically cisplatin-resistant cell lines. Loh et al. (Br. J. Cancer (1992) 66, 1109) confirmed these findings. Loh et al. furthermore came to the conclusion that the increased accumulation of satraplatin, which is a result of its enhanced lipophilicity, accounts for the dramatic increase of the potency of satraplatin over cisplatin. Other studies reporting on the activity of satraplatin towards cell lines with acquired or intrinsic resistance to cisplatin are those of Mellish et al. (Br J Cancer (1993) 68, 240), using human cervical squamous cell carcinoma cell lines, and Orr et al. (Br J Cancer (1994) 70, 415), using murine leukaemia cell lines. In the latter report the cell lines used were not just resistant to cisplatin, but also to tetraplatin and carboplatin.

Furthermore, cisplatin was repeatedly shown not to be effective against prostate cancer. Qazi & Khandekar (Am J Clin Oncol (1983) 6, 203) demonstrated in a phase II trial that cisplatin is not effective in patients with metastatic prostatic carcinoma. Hasegawa et al. (Cancer & Chemother (1987) 14, 3279) reported that the range of effective dose was wider for other platinum agents like carboplatin than for cisplatin. Even in combination treatment, cisplatin-comprising regimens demonstrate limited activity, e.g. in combination with mitoxantrone in metastatic prostate cancer (Osbome et al., Eur J Cancer (1992) 28, 477). Therefore, cisplatin is not a substitute for satraplatin as an agent to be used in prostate cancer.

Twentyman et al. (Cancer Res (1992) 52, 5674) investigated the sensitivity of human lung cancer cell lines with acquired or inherent resistance to cisplatin, to a series of novel platinum compounds, including satraplatin. In this study, cisplatin and carboplatin were found to act very similar, whereas satraplatin did not.

In spite of different routes of administration Kelland et al. (Int J Oncol (1993) 2, 1043) demonstrated the surprising finding that the efficacy of orally administered satraplatin is comparable to that of cisplatin and carboplatin administered intravenously, as determined in human ovarian carcinoma xenograft models. These findings were confirmed by Rose et al. (Cancer Chemother Pharmacol (1993) 32, 197), using murine and human tumor models. McKeage et al. (Cancer Res (1994) 54, 4118) investigated the differences of the schedule dependencies associated with these routes of administration.

In another study by Kelland et al. (Cancer Res (1993) 53, 2581) many of the above mentioned differences between satraplatin and cisplatin were confirmed. Furthermore it was found, that the cytotoxicity of satraplatin was dependent on the time of drug exposure. Again, it was confirmed that satraplatin does not exhibit cross resistance to cisplatin, whereas other platinum agents, e.g. tetraplatin, do. Without being bound to any particular theory, satraplatin circumvents transport-determined acquired resistance to cisplatin.

Mellish et al. (Cancer Res (1994) 54, 6194) investigated the mechanisms of acquired resistance to satraplatin in two human ovarian carcinoma cell lines. They found that, in contrast to cisplatin, acquired resistance to satraplatin is not mediated through reduced drug accumulation, but by increased intracellular GSH levels or increased DNA repair.

Sharp et al. (Clin Cancer Res(1995) 1, 981) compare the transport of cisplatin and satraplatin in human ovarian carcinoma cell lines. Cisplatin transport in the parental cell lines occurs via passive diffusion and active/facilitated transport, whereas in a cisplatin-resistant cell lines cisplatin enters cells by passive diffusion only. Without being bound to any particular theory, satraplatin circumvents cisplatin resistance by increasing the drug uptake. The mechanism of satraplatin transport across cell membranes is through passive diffusion, predominantly as a result of its enhanced lipophilicity.

Fink et al. (Cancer Res (1996) 56, 4881) investigated the effect of the loss of DNA mismatch repair activity on the sensitivity to cisplatin, satraplatin and other platinum agents. In contrast to cisplatin and carboplatin, which form the same type of adducts in DNA, there was no difference in sensitivity between mismatch repair-proficient and mismatch repair-deficient cell lines for satraplatin.

Perego et al. (Mol Pharmacol (1998) 54, 213) investigated the sensitivity of strains of *Schizosaccharomyces pombe* to cisplatin, satraplatin and other platinum compounds. The panel of the 23 yeast strains tested comprised many mutants in genes that affect the response to radiation. Whereas the mutants fell into three groups with respect to their sensitivity to cisplatin (minimal change in sensitivity, hypersensitivity, and marked hypersensitivity), none of the mutants demonstrated an appreciable change in sensitivity to satraplatin.

Leyland-Jones et al. (Amer J. Pathol (1999), 155, 77) investigated genomic imbalances associated with acquired resistance to platinum analogues. Using three ovarian carcinoma cell lines they identified differences between the three platinum compounds cisplatin, satraplatin and AMD473 (picoplatin).

Amorino et al. (Int J Radiation Oncol Biol Phys (1999), 44, 399) investigated radiopotentiation by satraplatin and the role of repair inhibition. They found that satraplatin can potentiate the effects of radiation in human lung cancer cells, and that the mechanism of this effect is probably inhibition of DNA repair by satraplatin. Differences to other platinum drugs like cisplatin and carboplatin are indicated.

Vaisman et al. (Biochemistry (1999), 38, 11026) reported on the effects of DNA polymerases and high mobility group protein 1 on the carrier ligand specificity for translesion synthesis past platinum-DNA adducts, with respect to different platinum compounds.

Screnci et al. (Br J Cancer (2000) 82, 966) investigated the relationship between hydrophobicity, reactivity, accumulation and peripheral nerve toxicity of a series of platinum compounds. According to Screnci et al. the hydrophilicity of platinum drugs correlates with platinum sequestration in the peripheral nervous system, but not with neurotoxicity.

Wei et al. (J Biol Chem (2001) 276, 38774) reported on the effect of ligands on the specific recognition of intrastrand platinum-DNA cross-links by high mobility group box and TATA-binding proteins, with respect to different platinum compounds.

Fokkema et al. (Biochem Pharmacol (2002) 63, 1989) analysed in detail the satraplatin-, JM-118-, and cisplatin-induced cytotoxicities in relation to various parameters like platinum-DNA adduct formation, glutathione levels and p53 status in human tumor cell lines with different sensitivities to cisplatin. It was confirmed that satraplatin and JM-118 can partially circumvent intrinsic and acquired resistance to cisplatin. At equimolar basis, satraplatin induced lower levels of platinum-DNA adducts in the cell lines tested compared to cisplatin.

Taken together, fundamental differences exist between satraplatin and other platinum agents, such as cisplatin. These differences are the basis, lead to or play a role in many of the different characteristics of satraplatin, including different pharmacokinetic properties, different efficacy, a different toxicology profile, different ADME properties and different mechanisms that lead to drug resistance, only to name a few.

Docetaxel (tradename taxotere) was first approved for use in locally advanced or metastatic breast cancer in 1996. Docetaxel is also indicated, in combination with cisplatin, for the treatment of patients with unresectable, locally advanced or metastatic non-small cell lung cancer (NSCLC) who have not previously received chemotherapy for this condition, and as a single agent for patients with locally advanced or metastatic NSCLC after failure of prior platinum-based chemotherapy. Furthermore, docetaxel in combination with prednisone is indicated for the treatment of patients with HRPC.

A phase I study was conducted to determine the dose limiting toxicity, maximum tolerated doses and to recommend phase II doses of the combination of satraplatin and paclitaxel (Invest New Drugs (2002) 20, 55). Patients received paclitaxel intravenously over one hour on day 1 of each cycle. Oral satraplatin was administered on days 1-5 after the paclitaxel infusion. It was not an object of this study to assess the efficacy of the satraplatin /paclitaxel combination.

The combined anticancer effects of docetaxel and cisplatin were assessed in vitro in cell lines (Cancer Sci (2004) 95, 679). Treatment of cell lines with docetaxel for 24 hours followed by incubation with cisplatin showed a synergistic effect. In these experiments docetaxel followed by cisplatin showed a stronger antitumor effect than cisplatin followed by docetaxel. However, in this study only gastric cell lines were analysed. Furthermore, not all cell lines tested showed the same effect, and the report does not suggest the therapeutic utility of the findings.

### Summary of the invention

This present invention relates to the prevention and/or treatment of a cancer or a tumor, and in particular to a combination therapy, corresponding uses and compositions, and kits comprising docetaxel and a compound of formula Ia or formula II as active ingredients.

One object of the invention is the use of one of the active ingredients in the manufacture of a pharmaceutical for use, in combination with the other active ingredient, in the treatment of a cancer or a tumor.

Another object of the invention is to provide a therapeutic combination for the treatment of a cancer or a tumor.

Yet another object of the invention is to provide a pharmaceutical composition for use or the treatment of a cancer or a tumor.

Also described herein is a method of killing or inhibiting the growth of a tumor cell comprising contacting said cell with an effective amount of said combination of active ingredients.

Further described herein is a method for treating an individual suffering from a tumor or a cancer, comprising administering to the individual an effective amount of said combination of active ingredients.

Further described herein is a packaged pharmaceutical comprising a pharmaceutical composition and instructions to administer an effective amount of one pharmaceutical composition to an individual suffering from a cancer or a tumor, prior to the administration of another, second pharmaceutical composition.

It is another object of the invention to provide kits having a combination of active ingredients, with or without pharmaceutically acceptable diluents and carriers, which may be effectively utilized together for carrying out the novel combination therapies of the invention.
The solution offered is based on the surprising discovery that taxanes are highly synergistic in combination with a platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 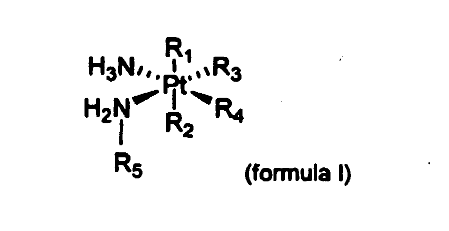 wherein R₁ and R₂ may be present or absent, each of R₁-F₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d). This synergistic effect is most pronounced, if docetaxel precedes the platinum-based chemotherapeutic agent.

### Brief description of the drawings

**Figure 1****.** Satraplatin (JM216) and certain of its metabolites according to Raynaud et al. (Cancer Chemother Phamacol (1996), 38, 155-162).
**Figure 2****.** Isobologram, indicating that satraplatin and docetaxel show an additive effect when administered simultaneously
**Figure 3****.** Isobologram, indicating that JM-118 and docetaxel show an additive effect when administered simultaneously
**Figure 4****.** Isobologram, indicating that docetaxel and satraplatin act synergistically when satraplatin is administered prior to docetaxel
**Figure 5****.** Isobologram, indicating that docetaxel and satraplatin act synergistically when docetaxel is administered prior to satraplatin
**Figure 6****.** Isobologram, indicating that docetaxel and JM-118 act synergistically when JM-118 is administered prior to docetaxel
**Figure 7****.** Isobologram, indicating that docetaxel and JM-118 act synergistically when docetaxel is administered prior to JM-118

### Detailed description of the invention

### 1. Definitions

The terms "administered", "administration", "administering" a compound will be understood to mean providing any compound of the methods of the invention to an individual in need of treatment.

The term "alkyl" refers to optionally substituted straight- or branched-chain saturated hydrocarbon groups having from 1 to about 20 carbon atoms, preferably from 1 to about 7 carbon atoms. Examples of alkyl include, but are not limited to, methyl, ethyl, n-propyl, i-propyl, n-butyl, s-butyl, t-butyl, n-pentyl, and s-pentyl. In addition, the term is intended to include both unsubstituted and substituted alkyl groups, the latter referring to alkyl moieties having one or more hydrogen substituents replaced by, but not limited to halogen, hydroxyl, carbonyl, alkoxy, ester, ether, cyano, phosphoryl, amino, imino, amido, sulfhydryl, alkythio, thioester, sulfonyl, nitro, heterocyclo, aryl or heteroaryl. It will also he understood by those skilled in the art that the substituted moieties themselves can be substituted as well when appropriate.

The term "cycloalkyl" refers to optionally substituted saturated cyclic hydrocarbon ring systems, preferably containing 3 to 7 carbons per ring. Exemplary groups include cyclopropyl, cyclobutyl, cyclopentyl, cyclohexyl, cycloheptyl, cyclooctyl, cyclodecyl, cyclododecyl, and adamantyl. Exemplary substituents include one or more alkyl groups as described above, or one or more of the groups described above as substituents for alkyl groups.

The term "effective amount" means the amount of the taxane or the subject compound, or the subject compound combination as defined below, that will elicit the biological, physiological, pharmacological, therapeutic or medical response of a cell, tissue, system, body, animal, individual, patient or human that is being sought by the researcher, pharmacologist, pharmacist, veterinarian, medical doctor, or other clinician, e.g., lessening of the effects/symptoms of cell proliferative disorders such as a cancer or tumor, or killing or inhibiting growth of a proliferating cell, such as a tumor cell.

The term "contacted", "contacting" and "brought into contact", refers to any process, method or route of administration, by which a drug, a compound, or any combination of drugs or compounds, is brought into vicinity with a target cell, such as a cancer cell or a cell derived from a tumor, in such a way that the drug, compound or combination of drugs or compounds, can exert its action onto said target cell. Said action on said target cell is typically a growth inhibitory, an anti-proliferative or a cytotoxic action.

The term "further treated", "further administer" or "further administered", means that the different therapeutic agents may be administered together, alternatively or intermittently. Such further administration may be temporally or spatially separated, for example at different times, on different days or via different modes or routes of administration.

The term "halogen" or "halo" refers to fluorine, chlorine, bromine and iodine.

The term "IC50", as used herein, refers to concentrations at which a measurable phenotype or response, for example growth of cells such as tumor cells, is inhibited by 50%. IC50 values can be estimated from an appropriate dose-response curve, for example by eye or by using appropriate curve fitting or statistical software. More accurately, IC50 values may be determined using non-linear regression analysis.

As used herein, an "individual" means a multi-cellular organism, for example an animal such as a mammal, preferably a primate. In addition to primates, such as humans, a variety of other mammals can be treated according to the method of the present invention. For example, mammals including, but not limited to, cows, sheep, goats, horses, dogs, cats, guinea pigs, rats or other bovine, ovine, equine, canine, feline, rodent or murine species can be used.

The term "metabolite", as used herein, refers to any substance produced by metabolism or by a metabolic process. Metabolism, as used herein, refers to the various physical/chemical/biochemical/phamacological reactions involved in the transformation of molecules or chemical compounds occurring in the cell, tissue, system, body, animal, individual, patient or human therein.

The term "prodrug", as used herein, refers to an agent which is converted into a pharmacologically active parent drug in vivo. Prodrugs are often useful because, in some situations, they may be easier to administer than the parent drug. They may, for instance, be bioavailable by oral administration whereas the parent drug is hot. The prodrug may also have improved solubility in pharmaceutical compositions over the parent drug. A prodrug may be converted into the parent drug by various mechanisms, including enzymatic processes and metabolic hydrolysis. See Gangwar et al., "Prodrug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999).

As used herein, a "proliferative disorder" includes a disease or disorder that affects a cellular growth, differentiation, or proliferation process. As used herein, a "cellular growth, differentiation or proliferation process" is a process by which a cell increases in number, size or content, by which a cell develops a specialized set of characteristics which differ from that of other cells, or by which a cell moves closer to or further from a particular location or stimulus. A cellular growth, differentiation, or proliferation process includes amino acid transport and degradation and other metabolic processes of a cell. A cellular proliferation disorder may be characterized by aberrantly regulated cellular growth, proliferation, differentiation, or migration. Cellular proliferation disorders include tumorigenic diseases or disorders. As used herein, a "tumorigenic disease or disorder" includes a disease or disorder characterized by aberrantly regulated cellular growth, proliferation, differentiation, adhesion, or migration, which may result in the production of or tendency to produce tumors. As used herein, a "tumor" includes a benign or malignant mass of tissue. Examples of cellular growth or proliferation disorders include, but are not limited to, cancer, e.g., carcinoma, sarcoma, or leukemia, examples of which include, but are not limited to, colon, ovarian, lung, breast, endometrial, uterine, hepatic, gastrointestinal, prostate, and brain cancer; tumorigenesis and metastasis; skeletal dysplasia; and hematopoietic and/or myeloproliferative disorders.

### 2. Platinum-based compounds

The subject platinum-based chemotherapeutic agents described herein are selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 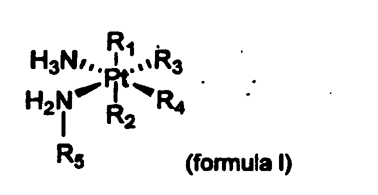 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d).

In one embodiment, the subject platinum-based compound is an orally available platinum chemotherapeutic agent. The phrase "orally available", as used herein, means that the drug or agent has biological, physiological, pharmacological, therapeutic, medically or clinically significant activity when administered orally. Suitable orally available platinum-based therapeutic agents include: satraplatin (JM216), JM118 and JM383 or a pharmaceutically acceptable salt, isomer or prodrug thereof, and others described in EP 0147926 and U.S. 5,072,011. However, it should be recognised that although a platinum-based chemotherapeutic agent may be orally available, such agent may also be administered through other appropriate routes, such as rectal, intramuscular, intravenous, intraperitoneal, and subcutaneous, which administration would still be recognised as following the teaching of the instant invention.

In another embodiment, the platinum-based compound is a platinum (IV) co-ordinated compound, in which the oxidation state of the platinum is +4. Examples are satraplatin (JM216), JM518, JM559, JM383, iproplatin, tetraplatin (ormaplatin), LA-12 ((OC-6-43)-bis(acetato)(1-adamantylamine)amminedichloroplatinum(IV)), JM149, JM221, JM335, ZD0473 (AMD473) and the platinum (Pt) IV compounds disclosed in US 6,413,953, US 5,072,011, US 5,519,155, US 5,547,982, US 6,518,428, WO 01/76569 and WO 02/28871, and Coordination Chemistry Reviews (2002) 232, 49-67.

In a further embodiment, the platinum-based compound is a platinum compound of a structure represented by the following general formula (formula 1):

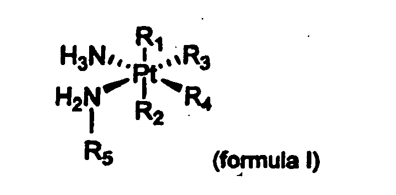

R1-R4 may be the same or different and are each independently selected from halogen, hydroxyl and acetate. R5 is a cycloalkyl, preferably a cyclohexyl. In certain embodiments, R1 and R2 are absent. In other embodiments, R1 and R2 are the same and are hydroxyl or acetate. In certain embodiments, R3 and R4 are the same and are both hydroxyl or preferably halogen, for example, chloride.

In a first aspect of the invention, the platinum-based chemotherapeutic agent is satraplatin. Satraplatin (JM216) has the structure:

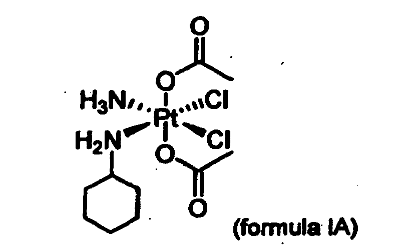

In another aspect of the invention, the platinum-based chemotherapeutic agent is JM-118. JM-118 has the structure:

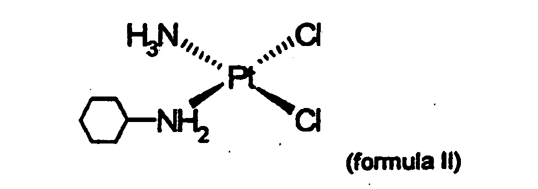

Satraplatin can be synthesised according to the method disclosed in U.S. Patent No. 5,072,011 and 5,244,919 or by appropriate modification of the method disclosed in US 6,518,428.

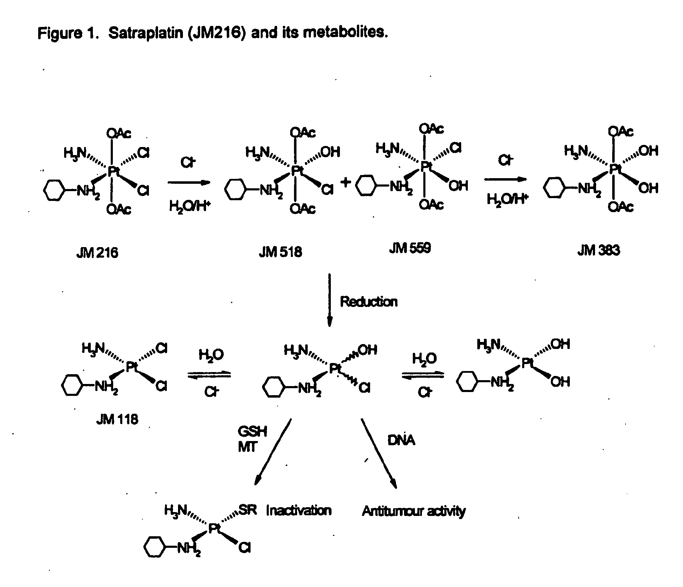

Upon administration of satraplatin to a cell, animal or a human patient, a number of related platinum-containing metabolites may be formed. The term "metabolite", as used herein, also includes a substance derived from a drug by physical, chemical, biological or biochemical processes in the body or cell after the drug is administered. Figure 1 (taken from Raynaud et al. 1996 Cancer Chemother Phamacol 38: 155-162) shows exemplary metabolites of satraplatin (JM216), and depicts JM118, JM383, JM518, JM559 and JM149. As will be appreciated by a person skilled in the art, additional platinum-containing molecules may be formed by metabolism of satraplatin after administration to a cell, animal or human patient, and such metabolites of satraplatin are encompassed in the scope of the instant invention. Suitable metabolites may be formed within the treated cell, animal or human by biological or biochemical biotransformation. Alternatively, such metabolites may be first formed out of the treated cell (such as in the GI tract), or may be formed by synthetic reaction from suitable starting materials and administered directly to the cell, animal or human patient. For example, JM118 may be synthesised according to the method disclosed in EP 147926, GB 2,060,615 and U.S. 4,329,299, or may be formed by biotransformation from JM216 in a separate fermentation step.

The platinum-based compound may be selected from satraplatin (JM216), JM118 and JM383 or a prodrug thereof. The term "prodrug", as used herein, also includes a substance that can give rise to a pharmacologically active metabolite. The prodrug itself may or may not be active; for example, it may be an inactive precursor.

An exemplary subject platinum-based chemotherapeutic agent may be brought into contact, exposed to or administered directly to the cell, individual, animal or human patient. However, as will be evident from the discussion of metabolites, a first platinum-based compound may be brought into contact, exposed to or administered to a cell, following which an exemplary platinum-based chemotherapeutic agent may be formed by metabolism of the first platinum-based compound. Such first platinum-based compound so administered may be considered a 'prodrug' of the exemplary subject platinum-based chemotherapeutic agent. For example, JM518 may be considered a prodrug of JM118, as JM118 (an exemplary compound useful for the method of the invention) is formed by metabolism of JM518. Analogously, JM216 may also be considered a prodrug of JM118. Other compounds that when administered to a cell, animal, individual, patient or human, are converted (metabolised) to an exemplary compound useful for the methods of the invention, such as JM118, would be considered within the scope of the instant invention. Such other compounds, may include salts, esters or phosphates of the exemplary subject compound useful for the method of the invention, and following the disclosure of the instant invention, a person skilled in the art would be able to envision a number of appropriate such prodrug compounds.

The platinum-based compound may be an intermediate in the synthesis of satraplatin (JM216), JM118 and JM383. Exemplary intermediates include IP-118 (U.S. Patent No. 4,687,780), JM-118 (an intermediate for synthesizing satraplatin, EP 147926) and JM149 (EP 333351).

Furthermore, the platinum-based compound may be represented by one of the following general structures:
(A) Those disclosed in US 5,072,011, represented by the following general structure:
   1. A Pt(IV) anti-tumor complex of the formula 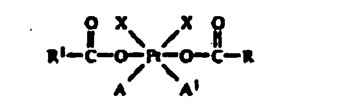
   wherein A and A¹ are individually selected from the group consisting of NH₃ and an amino group of I to 10 carbon atoms, with the proviso that when both A and A¹ are amino groups, at least one is an amino group of 1 to 3 carbon atoms; both X groups are the same and are Cl or Br; R and R¹ are individually selected from the group consisting of C₁-C₁₀ alkyl, cycloalkyl, aryl, aralkyl of 3 to 7 carbon atoms, alkoxy, alkenyl, alkylamino of 1 to 6 carbon atoms wherein the group is joined to the carbonyl through the hetero-atom in the case of alkoxy and alkylamino, and H; such that the X groups are cis to each other and the CO₂R and CO₂R¹ groups are trans to each other.
(B) Those disclosed in US 5,244,919, represented by the following general structure: 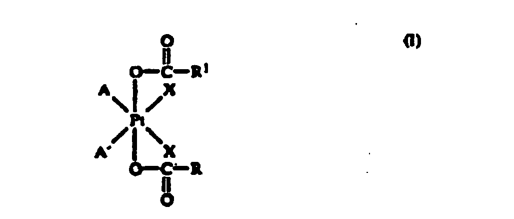 wherein A and A¹ are selected from the group consisting of NH_{*NH*3} and an amino group; R and R¹ are hydrogeo, C₁-C₁₀ alkyl, alkenyl, aryl, aralkyl, alkylamino or alkoxy; and X is halogen or alkyl monocarboxylate or dicarboxylate.
(C) Those disclosed in US 5,519,155, as represented by the general structure:
   1. A Pt(IV) complex of general formula I. 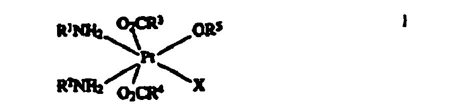 in which
      X is a halide atom, a pseudohalide, or hydroxy group,
      R¹ and R² are hydrogen, C₁ to C₆ straight or branched chain alkyl or cyclo-alkyl, aryl or R¹NH₂ is a heterocyclic nitrogen donor, and R¹ and R² may be the same as or different from one another,
      R⁹ and R⁴ are hydrogen, C₁ to C₃ straight or branched chain alkyl or cyclo-alkyl or aryl, and R³ and R⁴ may be the same as or different from one another, and p1 R⁵ is hydrogen, methyl or ethyl,
      and having the cis, trans, cis structure.
(D) Those disclosed EP 0 147 926 A1, as represented by the general structure: 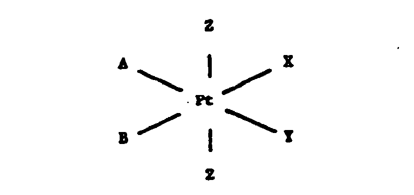 in which A and B are the sane or different and are each selected from amine and alkylamines or together represent a diaminocycloalkane. X and Y are the same or different and are selected from halide and pseudohalide or together represent cycloalkanedicarborylate, with the provisos that when X and Y together represent cycloalkanedicarboxylate A and B do not represent ammine and/or alkylamine, when A and B together represent a diaminocycloxexane X and Y do not represent halide and/or pseudohalide, are when A represents ammine B does not represent ethylamine, isopropylamine or cyclopentylamine and the Z moieties are optional and are selected from halide and hydroxy.
(E) Those disclosed in US 5,547,982 as represented by the general structures: 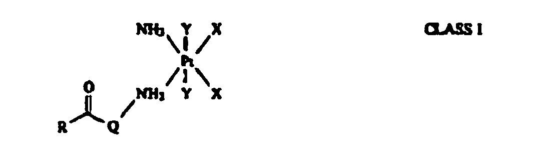 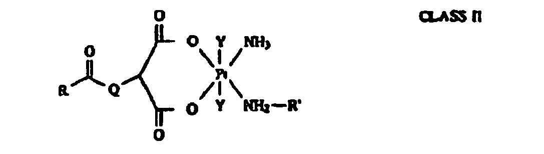 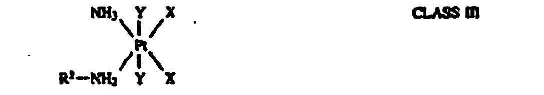 wherein R is H, lower alkyl of up to 8 carbons, alkenyl or alkynyl of up to 8 carbons or aryl; X is Cl, malonate, glycolate or oxalate; Y is OH, Cl, COOR¹ Lens B, or absent; Q is an alkylene, alkenyl, alkynyl or aryl linking group; R¹ is H, lower alkyl or aryl; R⁴ is H, aliphatic, aromatic or cyclo aliphatic group and R² is a cyclic aliphatic ketone, ketal**,** hemiacetal or acetal.
(F) Those disclosed in EB 0 727 430B1 as represented by the general structures: 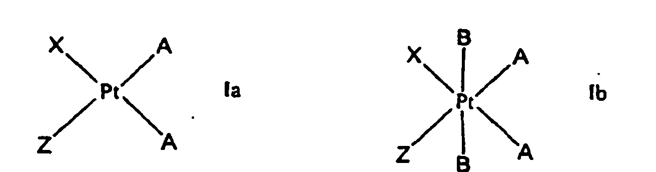 where
   each A is a leaving group and may be the same or different, or together form a bi-dentate carboxylate or
   each B. which may be the same or different, is halo, hydroxy, carboxylate, carbamate or carbonate ester. Z is a substituted amine wherein the substituent sterically hinders access of the Pt atom to a DNA strand of a tumor cell, wherein Z is an unsaturated cyclic amine coordinated to Pt through the amine nitrogen atom, which cyclic amine may contain one or more other heteroatoms and wherein said Z has a substituent on the atom adjacent the amine nitrogen atom and
   X is NH₃.
(G) Those disclosed in US 4,329,299 as represented by the general structures: 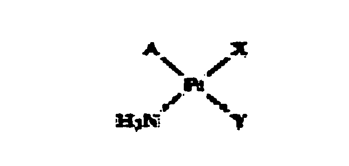 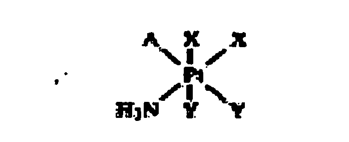
in which A is an amine having the formula R-NH2 where R is branched chain alkyl, and X and Y are the same or different halogen.

The platinum-based compounds described above will be collectively referred herein as the "subject platinum-based compounds" or "subject platinum-based chemotherapeutic agents". The subject platinum-based compounds also encompass any such compounds in pharmaceutically acceptable salt forms. The subject platinum-based compounds may contain one or more asymmetric centers, preferably carbon or platinum, and thus occur as geometrical isomers or stereoisomers. The present invention encompasses all these isomers and mixtures thereof, as well as pharmaceutically acceptable salts and prodrugs of the subject platinum-based compounds of formula IA or formula II.

### 3. Taxanes

Taxanes exert their cytotoxic effect by binding to tubulin, thereby causing the formation of unusually stable microtubules. The ensuing mitotic arrest triggers the mitotic spindle checkpoint and results in apoptosis. Other mechanisms that mediate apoptosis through pathways independent of microtubule dysfunction have been described as well, including molecular events triggered by the activation of Cell Division Control-2 (cdc-2) Kinase, phosphorylation of BCL-2 and the induction of interleukin 1β (IL-1 β) and tumor necrosis factor-α (TNF- α). Furthermore, taxanes have been shown to also exert anti-tumor activity via mechanisms other than the direct activation of the apoptotic cascade. These mechanisms include decreased production of metalloproteinases and the inhibition of endothelial cell proliferation and motility, with consequent inhibition of angiogenesis.

By the term "taxane", it is meant to include any member of the family of terpenes, including, but not limited to paclitaxel (Taxol) and docetaxel (Taxotere), which were derived primarily from the Pacific yew tree, *Taxus brevifolia*, and which have activity against certain tumors, particularly breast, lung and ovarian tumors (See, for example, Pazdur et al. Cancer Treat Res. 1993.19:3 5 1; Bissery et al. Cancer Res. 1991 51:4845). In the methods, uses, pharmaceutical compositions, packaged pharmaceuticals and kits described herein, preferred taxanes are paclitaxel, docetaxel, deoxygenated paclitaxel, TL-139 and their derivatives. See Annu. Rev. Med. 48:353-374 (1997).

The term "taxane" as used herein includes both naturally derived and related forms and chemically synthesized terpenes or derivatives thereof, including deoxygenated paclitaxel compounds such as those described in U.S. Pat. Nos. 5,440,056 and 4,942,184 and that sold as TAXOL® by Bristol-Myers Oncology. Pactitaxel has been approved for clinical use in the treatment of refractory ovarian cancer in the United States (Markman et al., Yale Journal of Biology and Medicine, 64:583, 1991; McGuire et al., Ann. Intern. Med., 111:273, 1989). It is effective for chemotherapy for several types of neoplasms including breast (Holmes et al., J. Nat. Cancer Inst., 83:1797, 1991) and has been approved for treatment of breast cancer as well. It is a potential candidate for treatment of neoplasms in the skin (Einzig et al., Proc. Am. Soc. Clin. Oncol., 20:46, 2001) and head and neck carcinomas (Forastire et al. Sem. Oncol., 20:56, 1990). The compound also shows potential for the treatment of polycystic kidney disease (Woo et al, Nature, 368:750, 1994), lung cancer and malaria. Docetaxel (N-debenzoyl-N-tert-butoxycarbonyl-10-deacetyl paclitaxel) is produced under the trademark TAXOTERE® by Rhone-Poulenc Rorer S.A. In addition, other taxanes are described in "Synthesis and Anticancer Activity of Taxol other Derivatives," D. G. 1. Kingston et al., Studies in Organic Chemistry, vol. 26, entitled "New Trends in Natural Products Chemistry" (1986), Atta-ur-Rahman, P. W. le Quesne, Eds. (Elvesier, Amsterdam 1986), pp 219-235. Various taxanes are also described In U.S. Patent No. 6,380,405.

### 4. Assays for effectiveness of treatment

In one embodiment, the platinum-based compounds described herein kill tumor cells when administered in combination with a taxane. Viability of a tumor cell can be determined by any methods known in the art. For example, one may use the colorimetric cytotoxicity assay described for anticancer drug screening in Shekan et al., J. Natl. Cancer. Inst. 82: 1107-12 (1990). For another example, one may determine the viability of a tumor cell by contacting the cell with a dye and viewing it under a microscope. Viable cells can be observed to have an intact membrane and do not stain, whereas dying or dead cells having "leaky" membranes do stain. Incorporation of the dye by the cell indicates the death of the cell. A dye useful for this purpose is trypan blue.

The exemplary taxanes and the platinum-containing composition described herein, may induce apoptosis, a mode of cell death, in resistant tumor cells. Apoptosis is recognized by a characteristic pattern of morphological, biochemical and molecular changes. Cells going through apoptosis appear shrunken and rounded. They also can be observed to become detached from a culture dish in which they are maintained. The morphological changes Involve a characteristic pattern of condensation of chromatin and cytoplasm which can be readily identified by microscopy. When stained with a DNA-binding dye, e.g., H33258, apoptotic cells display classic condensed and punctuate nuclei instead of homogenous and round nuclei.

A typical characteristic of apoptosis is endonucleolysis, a molecular change in which nuclear DNA is initially degraded at the linker sections of nucleosomes to give rise to fragments equivalent to single and multiple nucleosomes. When these DNA fragments are subjected to gel electrophoresis, they reveal a series of DNA bands which are positioned approximately equally distant from each other on the gel. The size difference between the two bands next to each other is about the length of one nucleosome, i.e., 120 base pairs. This characteristic display of the DNA bands is called a DNA ladder and it indicates apoptosis of the cell. Apoptotic cells can also be identified by flow cytometric methods based on measurement of cellular DNA content, increased sensitivity of DNA to denaturation, or altered light scattering properties. These methods are well known in the art. It should be recognized however, that modes of programmed cell death, including apoptosis, may be following a number of mechanisms or show other phenotypes/properties to those described above. In such cases, these mechanisms may also be characterized, classified or considered as apoptosis".

Cytotoxicity may also be measured using the SRB assay according to Shekan et al (J Natl Cancer Inst (1990) 82, 1107-112), as described in the Examples.

Additional assays for cell viability are described in Chapter 15 of Handbook of Fluorescent Probes and Research Products (Molecular Probes Handbook).

In one embodiment, a method of killing or inhibiting the growth of a tumor cell is described comprising contacting said cell with an effective amount of (a) docetaxel, and (b) satraplatin or JM-118. The growth inhibition of said tumor cells can be either partial (slowing down cell growth) or complete inhibition (i.e., arresting cells at a certain point in cell cycle). Cell growth can be measured by any techniques known in the art. Such techniques include, for example, MTT assay (based on reduction of the tetrazolium salt 3, [4,5-dimethylthiazol-2-yl]-2,5-diphenytetrazolium bromide), and PicoGreen assay using the DNA-binding dye Picogreen, both of which are described in Torrance, et al., Nat. Biotech. 19:940-945 (2001). Other assays for cell proliferation/growth are described in Chapter 15 of Handbook of Fluorescent Probes and Research Products (Molecular Probes Handbook).

Progression of disease, cancer or tumor in response to treatment can be monitored using any standard technique known in the art. For example, tumor size can be monitored and assessed to see if tumor size reduction has occurred as a result of the treatment. Monitoring and assessment may be aided by a variety of means including biopsies, manual inspection, microscopy, whole or partial body imaging and scans, and various molecular-based diagnostic and prognostic methods including those that investigate tumor-specific markers or mutations.

### 5. Tumors and other proliferative disorders

The subject compound combination is useful to treat proliferative disorders. The term "proliferative disorder" is art-recognized and further includes a disorder affecting an animal in a manner which is marked by aberrant, or otherwise unwanted, proliferation of a subset of cells of an animal. Cancer and tumors are proliferative disorders. Cells comprising or derived from a tumor will generally be understood to be proliferating cells, typically a hyper-proliferating cell, and in other circumstances, a tumor cell may be dysplastic, or may have proliferated.

It will be apparent to a person skilled in the art, on reading the disclosure of the instant invention, that the methods, pharmaceutical compositions and packaged pharmaceuticals comprising the subject compound combination will be useful for the treatment of other proliferative disorders, or for killing or inhibiting proliferating cells including tumor cells.

Any tumors may benefit from treatment with the uses, pharmaceutical compositions, and kits of the present invention. Suitable tumors may be solid tumors, which are cancer of body tissues other than blood, bone marrow, or the lymphatic system, such as carcinomas, sarcomas and lymphomas. Suitable tumors may also be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, skin cancer, cervical cancer, ovarian cancer, lung cancer, gynaecological cancers, urologocal and male genital cancers, soft tissue sarcomas, cancer of the major digestive glands, cancer of the bile duct, gall bladder cancer, bladder cancer, testicular cancer, cancers of the central nervous system, bronchial cancer, small and non-small-cell lung cancer, gastric cancer, and head and neck cancer. In some embodiments prostate cancer may be hormone-refractory prostate cancer.

Suitable tumors may also be hematological tumors. Hematological tumors may be leukemia, such as Acute Myelogenous Leukemia (AML), Acute Lymphoblastic Leukemia (ALL), Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, and Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin Lymphoma, Non-Hodgkin Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Extramedullary myeloma, and Granulocytic Sarcomae.

The subject platinum-based compounds are also believed useful in treating other types of proliferative disorders, including, proliferative disorders which are characterized by benign indications. Such disorders may also be known as "cytoproliferative" or "hyperproliferative" in that cells are made by the body at an atypically elevated rate. Such disorders include, but are not limited to, the following: hemangiomatosis in new born, secondary progressive multiple sclerosis, chronic progressive myelodegenerative disease, neurofibromatosis, ganglioneuromatosls, keloid formation, Paget's disease of the bone, fibrocystic disease of the breast, Peronies and Duputren's fibrosis, restenosis and cirrhosis.

### 6. Combination therapy, pharmaceutical formulations and dosages

Described herein are various aspects relating to a method of prevention and/or treatment of a cancer or a tumor, and in particular to a combination therapy, methods, compositions and pharmaceutical packages comprising a taxane and a platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 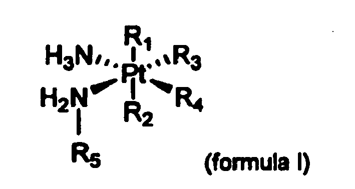 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d). Various methods, uses, therapeutic combinations, pharmaceutical compositions, packaged pharmaceuticals, formulations and kits are encompassed within the present disclosure which are based on this combination, and which are referred to as "subject combination therapy" or "subject compound combination".

The subject compound combination can be co-administered, e.g., in the same or different formulation. The term "co-administer" or "co-administered", as used herein, include administering two or more different therapeutic agents concurrently, sequentially or intermittently in all of the various aspects of the method described herein. Thus, the subject platinum-based compounds may be administered before, after, or together with a taxane to an individual in need thereof. The methods of the present invention can also be combined with other methods of cancer treatment, such as radiation therapy, surgery, or immunotherapy. In one embodiment the subject platinum-based compound is administered before the taxane. In another embodiment the taxane is administered before the subject platinum-based compound.

As shown in the Examples, Applicants have demonstrated that exemplary subject platinum-based compounds, including satraplatin (JM216) and JM-118, in combination with taxanes, including docetaxel, act highly synergistic In particulary when administered in a certain order. Thus, one embodiment described herein relates to methods of treating an individual suffering from a tumor or a cancer by administering to the individual an effective amount of (a) docetaxel, and (b) satraplatin or JM-118. In certain embodiments docetaxel is administered first. In other embodiments satraplatin or JM-118 is administered first.

In other embodiments, methods are described for killing or inhibiting the growth of a tumor cell comprising contacting said cell with an effective amount of (a) a taxane, and (b) a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 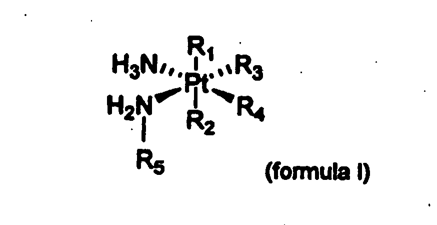 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d). In particular embodiments said taxane is docetaxel and/or said subject platinum-based chemotherapeutic agent is satraplatin or JM-118. In certain embodiments docetaxel is contacted with said cell first. In other embodiments satraplatin or JM-118 is contacted with said cell first.

Further described is the use of a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 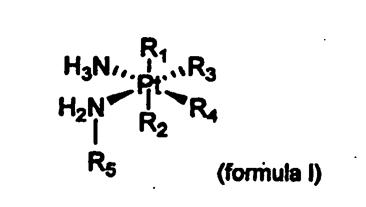 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d),
for the preparation of a first pharmaceutical composition for use in the treatment of an individual suffering from a cancer or a tumor, wherein said first pharmaceutical composition is administered within about 14 days of administration of a second pharmaceutical composition containing a taxane. In particular embodiments of the invention said taxane is docetaxel and said subject platinum-based chemotherapeutic agent is satraplatin or JM-118.

Further described is the use of a taxane for the preparation of a first pharmaceutical composition for use in the treatment of an individual suffering from a cancer or a tumor, wherein said first pharmaceutical composition is administered within about 14 days of administration of a second pharmaceutical composition containing a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 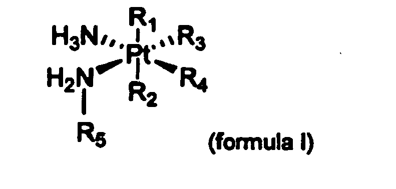 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutical acceptable salt, isomer or prodrug of (a) to (d). In particular embodiments of the invention taxane is docetaxel and said subject platinum-based chemotherapeutic agent is satraplatin or JM-118.

Further described is a for use in the treatment or prevention of a cancer or a tumor, wherein said taxane is administered with a subject platinum-based chemotherapeutic agent within about 14 days of each other. Further described is subject platinum-based chemotherapeutic agent for use in the treatment or prevention of a cancer or a tumor, wherein said subject platinum-based chemotherapeutic agent is administered with a taxane within about 14 days of each other. In particular embodiments of the invention said taxane is docetaxel and said subject platinum-based chemotherapeutic agent is satraplatin or JM-118.

Further described is a first pharmaceutical composition comprising a subject platinum-based chemotherapeutic agent as defined above, prepared according to the use described in the preceding paragraphs, included in a pharmaceutical package further including instructions to administer, to an individual suffering from a cancer or a tumor, said first pharmaceutical composition and said second pharmaceutical recited in the preceding paragraphs within about 14 days of each other.

Further described is a first pharmaceutical composition comprising a taxane as defined above, prepared according to the use described in the preceding paragraphs, included in a pharmaceutical package further including instructions to administer, to an individual suffering from a cancer or a tumor, said first pharmaceutical composition and said second pharmaceutical recited in the preceding paragraphs within about 14 days of each other.

Further described is a therapeutic combination for the treatment or prevention of a cancer or a tumor, including (a) a taxane and (b) a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 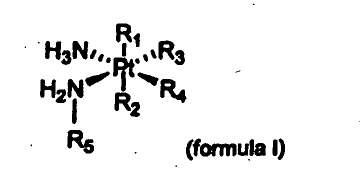 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d). In particular embodiments of the invention said taxane is docetaxel and said subject platinum-base chemotherapeutic agent is satraplatin or JM-118.

In other embodiment described herein a pharmaceutical composition is provided for the treatment or prevention of a cancer or a tumor, including (a) a taxane and (b) a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 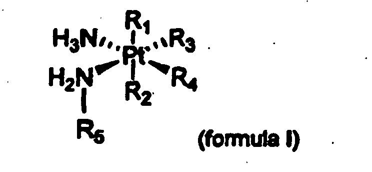 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d). In particular embodiments said taxane is dooetaxel and said subject platinum-based chemotherapeutic agent is satraplatin or JM-118.

Also described herein is a packaged pharmaceutical comprising a first pharmaceutical composition of a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 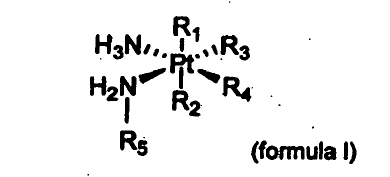 wherein R₁ and R₂ may be present or absent, each of R₁-R₄ is independently selected from halogen, hydroxyl, and acetate, and R₅ is a cycloalkyl;
(d) satraplatin or a metabolite of satraplatin; or a pharmaceutically acceptable salt, isomer or prodrug of (a) to (d),
wherein said packaged pharmaceutical further comprises instructions to administer, to an individual suffering from a cancer or a tumor, said first pharmaceutical composition and a second pharmaceutical composition containing a taxane within about 14 days of each other. In particular embodiments said taxane is docetaxel and said subject platinum-based chemotherapeutic agent is satraplatin or JM-118.

Also described herein is packaged pharmaceutical comprising a first pharmaceutical composition containing a taxane, wherein said packaged pharmaceutical further comprises instructions to administer, to an individual suffering from a cancer or a tumor, said first pharmaceutical composition and a second pharmaceutical composition containing a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 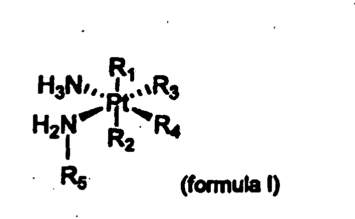 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d), within about 14 days of each other. In particular embodiments said taxane is docetaxel and said subject platinum-based chemotherapeutic agent is satraplatin or JM-118.

The packaged pharmaceutical may comprise instructions, or may provide otherwise, for the administration of one of said compounds to said individual at least 1 day, 2 days, 3 days, 5 days, 7 days, 10 days, or 14 days, before the other compound is administered to said individual. Hence, in certain embodiments said instructions provide for the sequential administration of the taxane and said subject platinum-based compound. In particular embodiments said taxane is administered to said individual 1 day, 2 days, 3 days, 5 days, 7 days, 10 days, or 14 days before said subject platinum-based compounds are administered to said individual. In other particular embodiments said subject platinum-based compound is administered to said individual 1 day, 2 days, 3 days, 5 days, 7 days, 10 days, or 14 days before said taxane is administered to said individual.

Further described is the use of a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 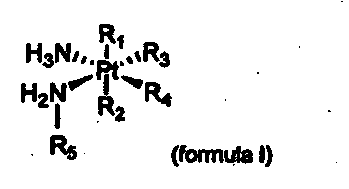 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d), in the manufacture of an anti-proliferative agent in a pharmaceutical package together with instructions for its use in combination with a taxane in the treatment of a cancer or a tumor. Also described is the use of a taxane in the manufacture of an anti-proliferative agent in a pharmaceutical package together with instructions for its use in combination with a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 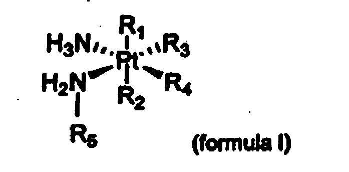 where in R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d), in the treatment of a cancer or a tumor. In particular embodiments of the invention said taxane is docetaxel and said subject platinum-based chemotherapeutic agent is satraplatin or JM-118.

The embodiments of the present invention provide for the sequential administration of the compounds, or the sequential contact or sequential exposure of a tumor, a cancer or a cell derived from or comprised in a tumor or a cancer, with the compounds of the present invention. For example, the phermaceutical compositions and the uses described herein provide for such sequential administration. Also, a tumor, a cancer or a cell derived from or being part of a tumor or a cancer may be brought in contact with, may be exposed to or may be treated via administration with a taxane at least 1 day, 2 days, 3 days, 5 days, 7 days, 10 days, or 14 days before a platinum-based chemotherapeutic agent is brought into contact with, is exposed to or is administered to said tumor, cancer or cell derived from or comprised in a tumor or a cancer. Likewise, a tumor, a cancer or a cell derived from or being part of a tumor or a cancer may be brought in contact with, may be exposed to or may be treated via administration with a platinum-based chemotherapeutic agent at least 1 day, 2 days, 3 days, 5 days, 7 days, 10 days, or 14 days before a taxane is brought into contact with, is exposed to or is administered to said tumor, cancer or cell derived from or comprised in a tumor or a cancer.

Further described is a kit for administering a first and a second pharmaceutical composition to an individual suffering from a cancer or a tumor, wherein said kit includes a plurality of separate containers, the contents of at least two containers differing from each other in whole or in part, wherein at least one of such containers contains a taxane, with or without additional pharmaceutical carrier or diluent, and at least one different container contains a subject platinum-based chemotherapeutic agent selected from:
(a) an orally available platinum-based chemotherapeutic agent;
(b) a platinum-based chemotherapeutic agent comprising a platinum (IV) co-ordination complex;
(c) a platinum-based chemotherapeutic agent represented by the following general structure: 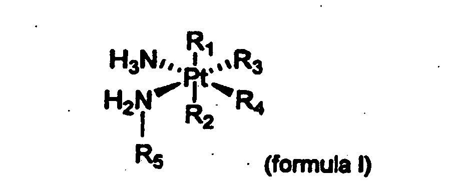 wherein R₁ and R₂ may be present or absent, each of R₁-R₄ is independently selected from halogen, hydroxyl, and acetate, and R₅ is a cycloalkyl;
(d) satraplatin or a metabolite of satraplatin;
or a pharmaceutically acceptable salt, isomer or prodrug of (a) to (d),
with or without additional pharmaceutical carrier or diluent.

In certain embodiments, the container of the kit containing a taxane does not contain a subject platinum-based chemotherapeutic agent, and/or the container of the kit containing a subject platinum-based chemotherapeutic agent does not contain a taxane.

In yet other embodiments, the container of the above kit containing a taxane and the container of the above kit containing a subject platinum-based chemotherapeutic agent are amongst, or represent, the at least two containers differing from each other in respect of their content in whole or in part.

In certain embodiments the kit further comprises instructions to administer, to an individual suffering from a cancer or a tumor, a first pharmaceutical composition containing a taxane and a second pharmaceutical composition containing a subject-platinum-based chemotherapeutic agent within about 14 days of each other. In particular embodiments of the invention said taxane is docetaxel and said subject platinum-based chemotherapeutic agent is satraplatin or JM-118. In certain embodiments said first and said second pharmaceutical composition are administered within 1 day, 2 days, 3 days, 5 days, 7 days, 10 days or 14 days of each other.

The present invention also provides for a pharmaceutical composition, use or kit as defined above, wherein said administration:
(i) is the sequential administration to said individual of a taxane as defined above and a subject platinum-based chemotherapeutic agent as defined above within about 14 days of each other; or
(ii)results in the sequential contact of a cell included in, derived from or being part of said cancer or tumour with a taxane as defined above and a subject platinum-based chemotherapeutic agent as defined above within about 14 days of each other.

Furthermore, the present invention also provides for a pharmaceutical composition use or kit as defined above, wherein said administration:
(i) is the sequential administration to said individual of first a taxane as defined above and then a subject platinum-based chemotherapeutic agent as defined above within about 14 days of each other;
(ii) results in the sequential contact of a cell included in, derived from or being part of said cancer or tumor with first a taxane as defined above and then a subject platinum-based chemotherapeutic agent as defined above within about 14 days of each other;
(iii) is the sequential administration to said individual of first a subject platinum-based chemotherapeutic agent as defined above and then a taxane as defined above within about 14 days of each other; or
(iv) results in the sequential contact of a cell included in, derived from or being part of said cancer or tumor with first a subject platinum-based chemotherapeutic agent as defined above and then a taxane as defined above within about 14 days of each other.

In particular embodiments of the invention said taxane is docetaxel and said subject platinum-based chemotherapeutic agent is satraplatin or JM-118.

The present invention also provides for a pharmaceutical composition, use or kit as defined above, wherein said administration:
(i) is the sequential administration to said individual of a taxane as defined above and a subject platinum-based chemotherapeutic agent as defined above within about 10 days, 7 days, 5 days, 3 days, 2 days or 1 day of each other; or
(ii) results in the sequential contact of a cell included in, derived from or being part of said cancer or tumour with a taxane as defined above and a subject platinum-based chemotherapeutic agent as defined above within about 10 days, 7 days, 5 days, 3 days, 2 days or 1 day of each other.

The present invention also provides for a pharmaceutical composition, use or kit as defined above, wherein said administration:
(i) is the sequential administration to said individual of a taxane as defined above and a subject platinum-based chemotherapeutic agent as defined above within about 48 hours, 24 hours, 12 hours, 8 hours, 6 hours, 4 hours, 2 hours, 1 hour, 30 mins, 15 mins or 5 mins of each other; or
(ii) results in the sequential contact of a cell included in, derived from or being part of said cancer or tumour with a taxane as defined above and a subject platinum-based chemotherapeutic agent as defined above within about 48 hours, 24 hours, 12 hours, 8 hours, 6 hours, 4 hours, 2 hours, 1 hour, 30 mins, 15 mins or 5 mins of each other.

It is further described that the first and second pharmaceutical compositions are administered to said individual effectively at the same time. In particular embodiments said taxane is docetaxel and/or said subject platinum-based chemotherapeutic agent is satraplatin or JM-118.

At is described that said platinum-based chemotherapeutic agent is administered orally. In particular embodiments of the invention said platinum-based chemotherapeutic agent is satraplatin or JM-118. It is further described that said taxane is administered intravenously. In particular embodiment of the invention, said taxane is docetaxel. Most preferably, satraplatin or JM-118 is administered orally and docetaxel is administered intravenously. These preferred routes of administration pertain to all uses, pharmaceutical compositions and other aspects of the present invention.

Further described is a packaged pharmaceutical comprising a first pharmaceutical composition and instructions to administer, to an individual suffering from a cancer or a tumor, said first pharmaceutical composition and a second pharmaceutical composition, wherein:
(i) the first or second pharmaceutical composition contains a taxane as defined above;
(ii) the other pharmaceutical composition is a subject platinum-based chemotherapeutic agent as defined above; and
(iii) said administration results in sequential contact of said subject platinum-based chemotherapeutic agent and said taxane with a cell included in, derived from or being part of the cancer or tumor of said individual, within 14 days of each other. In particular embodiments said taxane is docetaxel and or said subject platinum-based chemotherapeutic agent is satraplatin or JM-118.

The subject compound combination can be formulated and administered to treat individuals with cancer by any means that produces contact of the active ingredients with the agent's site of action in the body of a mammal. They can be administered by any conventional means available for use in conjunction with pharmaceuticals, either as individual therapeutic active ingredients or in a combination of therapeutic active ingredients. They can be administered alone, but are generally administered with a pharmaceutical carrier selected on the basis of the chosen route of administration and standard pharmaceutical practice.

In particular embodiments, the administration of said pharmaceutical formulations leads to a situation, in which the subject platinum-based compound is in contact with the agent's site of action in the body of an individual, before the taxane is in contact with the agent's site of action in the body of an individual. In other particular embodiments the administration of said pharmaceutical formulations leads to a situation, in which the taxane is in contact with the agent's site of action in the body of an individual, before the platinum-based compound is in contact with the agent's site of action in the body of an individual.

Pharmaceutical compositions for use in accordance with the present invention may be formulated in conventional manner using one or more physiologically acceptable carriers or excipients. The pharmaceutical compositions of the invention can be formulated for a variety of routes of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous (i.m., i.v., i.p., and s.c. respectively). For injection, the pharmaceutical compositions of the invention can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

The term "preparation of a [first] pharmaceutical composition", refers to any process or method performed or required in the generation of a pharmaceutical composition which is ready to be administered to a patient or an individual in need thereof. This includes the manufacture of the pharmaceutical composition, the formulation of the pharmaceutical composition, packaging of the pharmaceutical composition, and other steps performed before the pharmaceutical composition is delivered, requested or made available to a pharmacist, doctor or nurse. It also includes methods and processes performed by the pharmacist, doctor or nurse prior to the administration of the pharmaceutical composition. This includes, for example, dissolving the pharmaceutical composition in an appropriate solvent for administration, e.g. injection, and other steps performed by such a person which aids, facilitates, makes possible or enables the administration of the pharmaceutical composition.

The most preferred administration route of the subject platinum-based composition is oral. In oral administration, the pharmaceutical compositions may take the form of, for example, unit dose-forms such as tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active agent. In certain embodiments such a controlled release of the active agent is preferred. In certain embodiments the controlled release leads to a situation, in which the subject platinum-based compound is in contact with the agent's site of action in the body of an individual, before the taxane is in contact with the agent's site of action in the body of an individual. In other embodiments the controlled release leads to a situation, in which the taxane is in contact with the agent's site of action in the body of an individual, before the subject platinum-based compound is in contact with the agent's site of action in the body of an individual. For buccal administration the therapeutic compositions may take the form of tablets or lozenges formulated in a conventional manner. For administration by inhalation, the compositions for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The pharmaceutical compositions may also be formulated as a depot preparation. In some embodiment it is preferred that such a formulation leads to a situation, in which the subject platinum-based compound is in contact with the agent's site of action in the body of an individual, before the taxane is in contact with the agent's site of action in the body of an individual. In other embodiment it is preferred that such a formulation leads to a situation, in which the taxane is in contact with the agent's site of action in the body of an individual, before the subject platinum-based compound is in contact with the agent's site of action in the body of an individual. Such long acting formulations may be administered by implantation (for example subcutaneously or intramuscularly) or by intramuscular injection. Thus, for example, the therapeutic compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the compositions of the invention are formulated into ointments, salves, gels, or creams as generally known in the art. A wash solution can be used locally to treat an injury or inflammation to accelerate healing. For oral administration, the therapeutic compositions are formulated into conventional oral administration forms such as capsules, tablets, and tonics.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton. The pack or dispenser device may further comprise instructions to first administer one of the compounds of the subject compound combination. In some embodiments said first compound to administer is a taxane. In other embodiments said first compound to administer is a subject platinum-based compound.

In certain particular embodiments the subject compound combination is formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566. The pharmaceutical compositions of the present invention can be used to provide slow or sustained release of one or more of the active ingredients. In certain embodiments the slow or sustained release of one or more of the active ingredients leads to a situation, in which the subject platinum-based compound is in contact with the agent's site of action in the body of an individual, before the taxane is in contact with the agent's site of action in the body of an individual. In other embodiments the slow or sustained release of one or more of the active ingredients leads to a situation, in which the taxane is in contact with the agent's site of action in the body of an individual, before the subject platinum-based compound is in contact with the agent's site of action in the body of an individual. In order to provide the desired release profile in varying proportions, hydropropylmethyl cellulose, other polymer matrices, gels, permeable membranes, osmotic systems, multilayer coatings, microparticles, liposomes, microspheres, or the like, or a combination thereof can be used. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

The pharmaceutical compositions of the present invention may be formulated in a neutral or salt form. Pharmaceutical-acceptable salts include the acid addition salts and are formed with inorganic acids such as, for example, hydrochloric or phosphoric acids, or such organic acids as acetic, oxalic, tartaric, mandelic, and the like. Salts formed with the free carboxy1 groups can also be derived from inorganic bases such as, for example, sodium, potassium, ammonium, calcium, or ferric hydroxides, and such organic bases as isopropylamine, trimethylamine, histidine, procaine and the like.

The subject compound combination can also be co-administered with a variety of other drugs. For example, the subject compound combination can be used as part of a regiment of treatment in which it is combined with other chemotherapeutic agents including anti-cancer therapeutic agents that inhibit cancer growth, anti-angiogenesis agents and anti-metastatic agents. The subject pharmaceutical compositions may also be combined with immunomodulators.

In a further embodiment, the subject compound combination is administered to a patient to whom an anti-emetic agent is also administered. Anti-emetic agents according to this invention include any anti-emetic agents known to the skill artisan, including, but not limited to, serotonin-3 receptor antagonists like granisetron, ondansetron and tropisetron, NK1 receptor antagonists, antihistamines such as cinnarizine, cyclizine and promethazine, histamine H2 receptor antagonists such as ranitidine (Zantac), phenothiazines such as chlorpromazine, droperidol, haloperidol, methotrimeprazine, perphenazine, trifluoperazine and prochlorperazine, domperidone, and metoclopramide.

In other embodiments, the subject compound combination is administered to a patient who is also treated with an anti-diarrheal such as loperamid, corticosteroide such as cortisone, growth hormone or growth factor such as GCSF or erythropoietin, a diuretica such as furosemid, steroidal or non-steroidal analgesics such as an opiate, e.g. morphine, or paracetamol or anti-hyperuricemics such as allopurinol.

In other embodiments, the subject compound combination is administered to a patient, who is also treated with thrombocytes, erythrocytes or whole blood.

In other embodiments, the subject compound combination is administered to a patient, who is also treated with stem cells of the bone marrow.

Other embodiments described herein relate to a method of therapeutic patient care. In the method, a patient who is treated via administration with the subject compound combination receives food parenterally.

The present invention additionally provides methods for preparing a pharmaceutical composition useful for the treatment of an individual suffering from a cancer or tumor. The methods comprise:
a) compiling data including:
   i. bioequivalence data for a compound combination comprising a taxane as defined above and a subject platinum-based chemotherapeutic agent as defined above, or a pharmaceutically acceptable salt, isomer or prodrug thereof, compared to a marketed originator compound or compound combination; or
   ii. clinical data demonstrating the effectiveness of said subject compound combination in treating cancer patients;
b) submitting said compiled data to a drug regulatory authority for the purpose or obtaining regulatory or marketing approval for said subject compound combination for the treatment of cancer patients; and
c) manufacturing, importing, packaging/re-packaging, labeling/re-labeling or marketing said subject compound combination, or license rights to said approval, for the treatment of cancer patients.

The dosage administered will be a therapeutically effective amount of the subject compound combination sufficient to result in amelioration of symptoms of the cancer or tumor and will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

Toxicity and therapeutic efficacy of pharmaceutical compositions of the present invention can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapeutic agents which exhibit large therapeutic indices are preferred. While therapeutic compositions that exhibit toxic side effects may be used, care should be taken to design a delivery system that targets such therapeutic agents to the site of affected tissue in order to minimize potential damage to uninfected cells and, thereby, reduce side effects.

The data obtained from cell culture assays and animal studies can be used in formulating a range of dosage for use in humans. The dosage lies preferably within a range of circulating concentrations that include the ED50 with little or no toxicity. The dosage may vary within this range depending upon the dosage form employed and the route of administration utilized. For any agents used as described herein, the therapeutically effective dose can be estimated initially from cell culture assays. A dose may be formulated in animal models to achieve a circulating plasma concentration range that includes the IC50 (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depends upon a number of factors known to those or ordinary skill in the art, e.g., a physician. The dose(s) of the small molecule will vary, for example, depending upon the identity, size, and condition of the subject or sample being treated, further depending upon the route by which the composition is to be administered, if applicable, and the effect which the practitioner desires the therapeutic to have upon the therapeutic target of targets, such as nucleic acid or polypeptide of the invention, through with the disease causes, symptoms or effects are mediated. As will be self-evident, for combination therapy the combined effect of both compounds, the taxane and the subject platinum-based compound, will have to be taken into account.

Exemplary doses include milligram or microgram amounts of the small molecule(s), i.e. the taxane and the subject platinum-based chemotherapeutic agent, per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram.

A person skilled in the art will appreciate that doses can also be calculated on a body surface basis. A person of 70 kg has an approximate body surface area of 1.8 square meter doses include milligram or microgram amounts of the small molecule per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

### 7. Treatment of resistant or refractory cancers and tumors.

Cancers or tumors that are resistant or refractory to treatment of a variety of anti-proliferative agents may benefit from treatment with the methods described herein. In certain alternative embodiments of the instant invention, the subject compound combination may be useful in treating tumors that are refractory or resistant to an anti-proliferative agent. In particular embodiments said anti-proliferative agent is not a hormone-based drug. In other particular embodiments said anti-proliferative agent is cisplatin. Resistance to anti-proliferative agents can be tested and verified using the methods described in the Examples.

As used herein, the term "anti-proliferative agent" relates to any compound which is or may be used in the treatment of a "proliferative disorder", as defined herein. Exemplary anti-proliferative agents include vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide), anti-mitotics, taxanes (paclitaxel, taxol derivatives, but not docetaxel), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine, nocodazole, platinum-based agents (cisplatin, paraplatin, carboplatin, but not the subject platinum-based chemotherapeutic agents as defined herein), camptothecin, 9-nitrocamptothecin (Orethecin, rubitecan), 9-aminocamptothecin (IDEC-13'), exatecan (DX-8951f), lurtotecan (GI-147211C), BAY 38-3441, the homocamptothecins such as diflomotecan (BN-80915) and BN-80927, topotecan (Hycamptin), NB-506, J107088, pyrazolo [1,5-a] indole derivatives, such as GS-5, lamellarin D, irinotecan (Camptosar, CPT-11), and antibodies, such as 1D10, Hu1D10, 1 D09C3, 1 C7277, 305D3, rituximab, 4D5, Mab225, C225, Daclizumab (Zenapax), Antegren, CDP 870, CMB-401, MDX-33, MDX-220, MDX-477, CEA-CIDE, AHM, Vitaxin, 3622W94, Therex, 5G1.1, IDEC-131, HU-901, Mylotarg, Zamyl (SMART M195), MDX-210, Humicade, LymphoCIDE, ABX-EGF, 17-1A, Trastuzumab (Herceptin ®, rhuMAb), Epratuzumab, Cetuximab (Erbitux ®), Pertuzumab (Omnitarg®, 2C4), R3, CDP860, Bevacizumab (Avastin ®), tositumomab (Bexxar ®), Ibritumomab tiuxetan (Zevalin ®), M195, 1D10,Hu1D10 (Remitogen®, apolizumab), Danton/DN1924, an "HD" antibody such as HD4 or HD8, CAMPATH-1 and CAMPATH-1H or other variants, fragments, conjugates, derivatives and modifications thereof, or other equivalent compositions with improved or optimized properties.

Refractory cancers or tumors include those that fail or are resistant to treatment with anti-proliferative agents alone, radiation alone or combinations thereof. For the purposes of this specification, refractory cancers or tumors also encompass those that appear to be inhibited by treatment with chemotherapeutic agents and/or radiation but recur up to five years, sometimes up to ten years or longer after treatment is discontinued.

The term "resistant", as used herein, include both partially resistant and completely resistant. Thus, a tumor that is only partially resistant to an anti-proliferative agent may nonetheless benefit from treatment with the subject compound combination. Indeed, in certain embodiments it may be beneficial to treat a tumor if such resistance is merely suspected, may not yet be know or even before such resistance has developed. In alternative embodiments, it may be subsequently determined, or not at all, that the cancer or tumor was resistant or refractory to an anti-proliferative agent.

In particular embodiments said anti-proliferative agent is not a hormone-based drug. In certain embodiments said anti-proliferative agent is not a pituitary down-regulator. In other embodiments said anti-proliferative agent is not an anti-androgen.

The term "hormone-based drug" refers to compounds which are used in hormonal treatment. Such compounds may be hormones or derivatives or variants of hormones. Hormone-based drugs also include molecules which are neither hormones, nor derivatives or variants of hormones, yet affect the production or action of hormones. Treatment with hormone-based drugs is referred to as "hormone ablation therapy". Hormone ablation therapy aims at limiting the growth of a cancer or tumor by limiting the supply of hormones that this type of cancer or tumor needs for growth.

Some types of cancer, e.g. cancer of the prostate, depend on hormones, e.g. testosterone, for growth. If the amount of testosterone is reduced it is often possible to slow down or shrink the tumour. Such treatment is usually effective for a limited time, typically for 18 to 24 months. After that, the tumor may stop responding to the treatment and resume growth, i.e. hormone refractory prostate cancer (HRPC) develops.

Testosterone levels can be reduced, for example, by surgery (e.g. removel of the testes) or by drug-based treatment, including hormone-based drug treatment. There are two main types of such hormone based drugs. First, pituitary down-regulators block luteinizing hormone-releasing hormone (LHRH), which is released by the pituitary gland. LHRH, if not blocked is a stimulus for the testes to produce testosterone. Examples of such pituitary down-regulators include leuprorelin (Prostap), triptorelin (De-capaptyl), buserelin (Suprefact) and goserelin (Zoladex). Second, anti-androgens block the action of testosterone at the prostate. Examples of such anti-androgens include cyproterone acetate (Cyprostat), flutamide (Eulexin, Drogenil), nilutamide (Nilandrone) and bicalutamide (Casodex). It will be appreciated that other types of cancer may also be treated with hormone-based drugs. These include, but is not limited to, breast cancer, uterine cancer, thyroid cancer and colon cancer.

The practice of aspects of the present invention may employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

While the invention has been described and exemplified in sufficient detail for those skilled in this art to make and use it, various alternatives, modifications, and improvements should be apparent

Those skilled in the art will recognize, or be able to ascertain using no more than routine experimentation, many equivalents to the specific embodiments of the invention described herein. Such equivalents are intended to be encompassed by the following claims. Those skilled in the art will also recognize that all combinations of embodiments or features of the claims described herein are within the scope of the invention.

### Exemplification

### Example 1. Efficacy of satraplatin and its metabolites is maintained in cisplatin-resistant tumor cells

In this comparative example, we observed the surprising finding that subject platinum-based compounds of the invention were useful in inhibiting or killing tumour cells that were resistant to other platinum compounds, such as cisplatin.

The A129 cp80 cell line (received from Tito Fojo, NIH; Biochem Pharmacol 52, 1855), derived from the ovarian carcinoma A2780, was highly resistant to cisplatin - relative resistance in individual experiments ranged between 80 to 106-fold - yet remained susceptible to treatment with JM216, JM118 and JM383 - relative resistance in individual experiments between 0.19 to 2.59-fold (Table 1). The parental non-mutated cell line A129 was used as control.

1,000-5,000 cells/well were contacted with the test compounds for 48 hours at various concentrations in order to calculate the IC50 values shown in Table 1. Cytotoxicity was measured using the SRB assay according to Shekan et al. (J Natl Cancer Inst (1990) 82, 1107-112). Briefly, cells were plated in 96 well dishes 24 hours prior to compound addition. The assay was terminated with the addition of cold TCA to a final concentration of 10% and the plates were incubated for one hour at 4°C. The plates were then washed 5 times with water and 100 µl of a Sulforhodamine B solution (4%) was added to each well. The plate was then incubated for 10 minutes at room temperature before removal of unbound dye by washing with 1% acetic acid. The bound dye was solubilized with 10 mM Trizma base and the absorbance read at OD570.

**Table 1. Cellular IC50's of Satraplatin and metabolites in Cisplatin resistant cells**

| Compound | Cell line | | RR |
|---|---|---|---|
| | A129 IC50 (µM) | A129 cp80 IC50 (µM) | |
| Cisplatin | 0.23 +/- 0.17 (3) | 15.1 +/- 6.4 (3) | 80 +/- 25 |
| Satraplatin | 0.30 +/- 0.24 (3) | 1.54 +/ 0.72 (3) | 5.9 +/- 1.9 |
| (JM216) | | | |
| JM118 | 0.24 +/- 0.14 (3) | 0.69 +/- 0.52 (3) | 2.6 +1- 0.9 |
| JM383 | 1.29 +/- 0.11 (2) | 1.78 +/- 1.15 (2) | 1.4 +/- 1.0 |

### Legend:

Table 1 shows the IC50 values as determined in the experiments described in Example 1. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the relative level of resistance conferred to the indicated drugs by the mechanism that confers cisplatin resistance.

### Example 2. Synergism between the subject platinum-based compounds and docetaxel.

We observed the surprising finding that the subject platinum-based compounds, in particular satraplatin and JM-118, act synergistically in combination with docetaxel, when exposed to or brought into contact with cancer cells or tumor cells, in sequential order.

The prostatic adenocarcinoma cell line PC-3 (ATCC order number: CRL-1435; Invest Urol (1979) 17, 16; Cytogenet Cell Genet (1993) 62,183) was used. PC-3 cells were harvested from sub-confluent plates and seeded in 96 well dishes at a density of 2,000 cells per well. The cells were cultured at 37°C, 5% CO₂ in F-12K media supplemented with 10% FCS and 1% Pen/Strep. Twenty-four hours after plating, the cells were contacted with a range of concentrations of the individual compounds and incubated for 48 hours. Then cytotoxicity was measured using the SRB assay according to Shekan et al. (Example 1; J Natl Cancer Inst (1990) 82, 1107-112).

For simultaneous exposure, cells were incubated with concentrations of satraplatin or JM-118 representing 0.0675x, 0.125x, 0.25x, 0.5x, 1x and 2x of the previously determined IC50s (4.0 and 0.82 µM, respectively, as determined with the SRB assay (see J Natl Cancer Inst (1990) 82, 1107-112 and example 1) and to the same relative concentrations of docetaxel (IC50 of 0.008 µM) at a constant ratio. The cells were incubated for 48 hours and the effect of this combination of compounds on cellular proliferation was determined with the SRB assay.

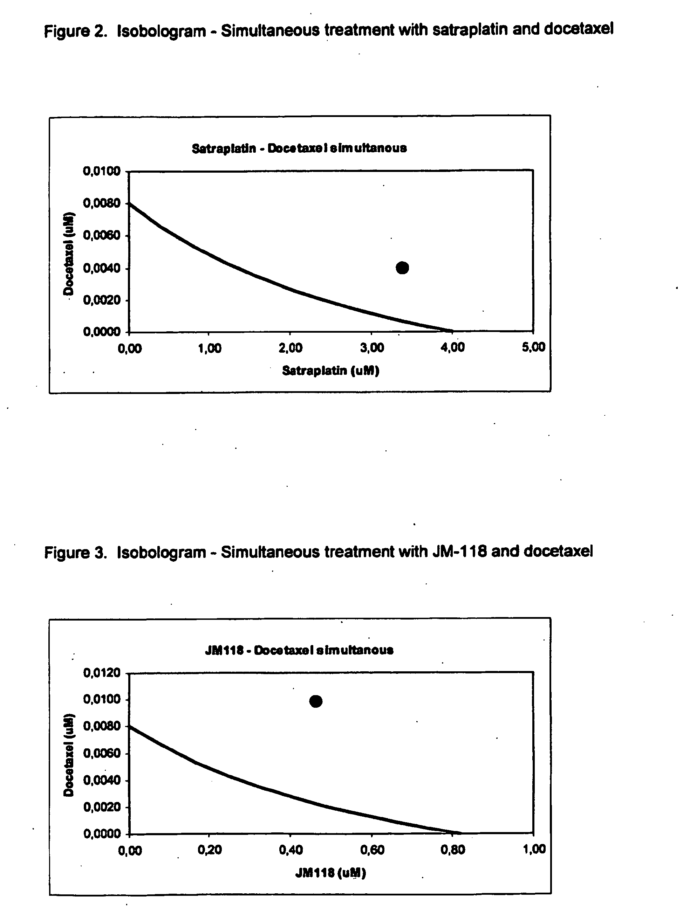

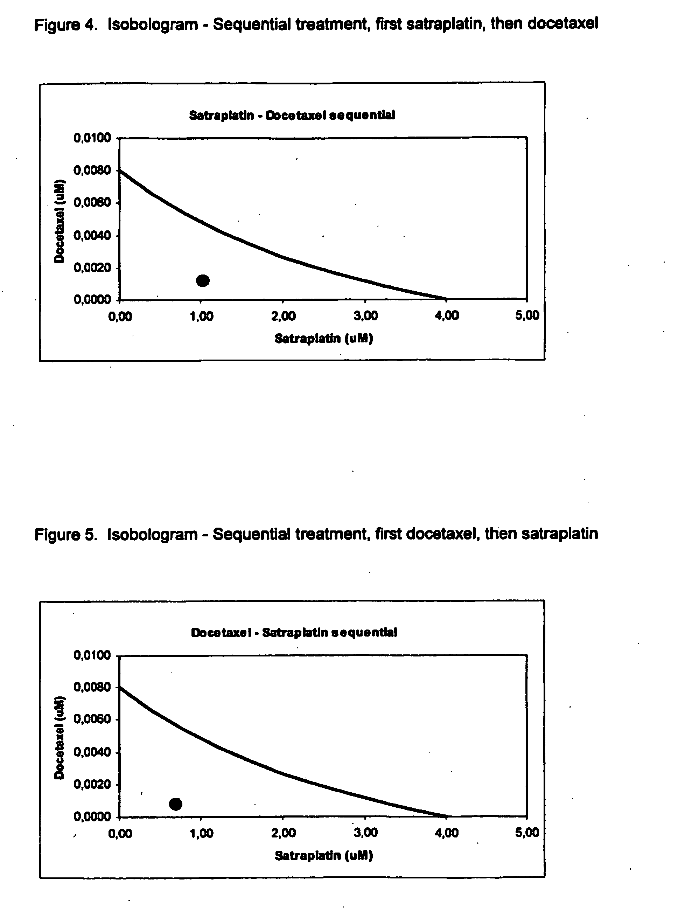

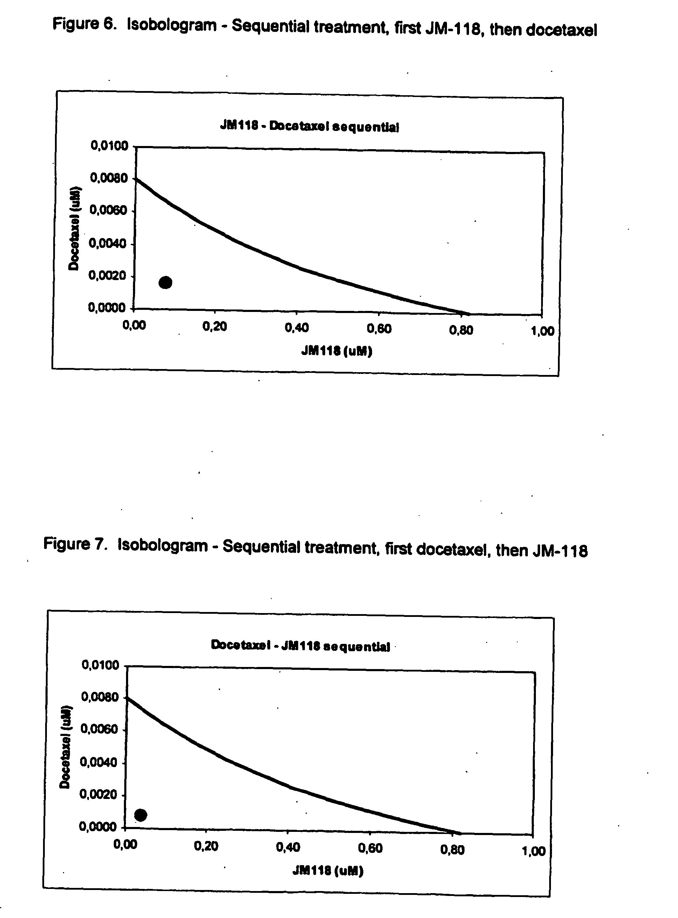

For sequential exposure, cells were incubated with either docetaxel for 48 hours followed by a wash step and contacted with satraplatin or JM-118 for an additional 48 hours or satraplatin or JM-118 followed by docetaxel. As above, the effect on cellular proliferation was determined with the SRB assay.
The combination index (Cl; Adv Enzyme Regul (1984) 22,27) was calculated with the algorithm of Chou using XLfit 4.1 (IDBS Ltd., Guildford, UK) with the effect levels which caused an inhibition of 50 %. Cl values of <1, ≈1 and >1 indicate synergism, additive effect and antagonism, respectively. The CI's for the combined effect of the two pairs of drugs following simultaneous addition or sequential exposure are summarized in Table 2. Figures 2-7 shows the isobolograms for all drug combinations tested. When exposed to the cells sequentially, the combination of satraplatin or JM-118 and docetaxel show a clearly and strong synergistic effect. In contrast, an additive effect was observed when drugs were added simultaneously.

**Table 2. Cl's of the two pairs of drugs following simultaneous addition or sequential exposure**

| **Combination** | **CI at 50 %** | **Result / Effect** |
|---|---|---|
| Satraplatin and docetaxel simultaneously | 1.34 | Additive |
| JM-118 and docetaxel simultaneously | 1.35 | Additive |
| Satraplatin first, then docetaxel | 0.40 | Synergistic |
| Docetaxel first, then satraplatin | 0.27 | Synergistic |
| JM-118 first, then docetaxel | 0.22 | Synergistic |
| Docetaxel first, then JM-118 | 0.11 | Synergistic |

### Legend:

Table 2 shows the Cl values as determined in the experiments described in Example 2. Cl values were calculated with the effect levels which caused an inhibition of 50 %. Both drug combinations, satraplatin and docetaxel, as well as JM-118 and docetaxel, were highly synergistic when added in sequential order. When added simultaneously, each pair of drugs had an additive effect.

## Claims

1. A first pharmaceutical composition containing either (i) a compound having the structure of: 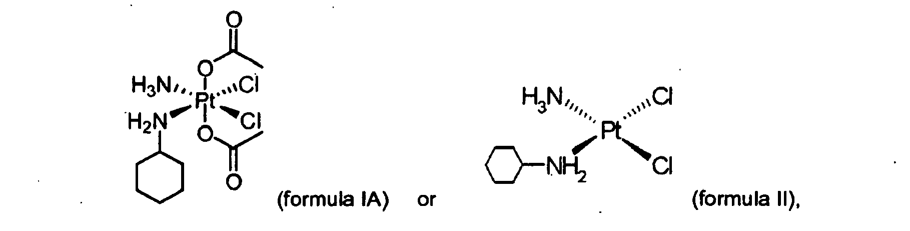 or (ii) docetaxel, wherein said first pharmaceutical composition is for use in treating an individual suffering from a cancer or a tumor by sequential administration of said first pharmaceutical composition and a second pharmaceutical composition containing, in the case of (i) docetaxel, or in the case of (ii) a compound as described in (i), within about 14 days of each other.

2. Use of either (i) a compound of Formula IA or Formula II, or (ii) docetaxel, for the preparation of a first pharmaceutical composition for the treatment of an individual suffering from a cancer or a tumor, wherein said first pharmaceutical composition is to be sequentially administered within about 14 days of administration of a second pharmaceutical composition containing, in the case of (i) docetaxel, or in the case of (ii) a compound of Formula IA or Formula II

3. A kit for administering a first and a second pharmaceutical composition to an individual suffering from a cancer or a tumor, wherein said kit includes a plurality of separate containers, the contents of at least two containers differing from each other in whole or in part, wherein at least one of such containers contains docetaxel, with or without additional pharmaceutical carrier or diluent, and at least one different container contains a compound of Formula IA or Formula II. with or without additional pharmaceutical carrier or diluent.

4. The kit according to claim 3, wherein the container containing docetaxel does not contain a compound of Formula IA or Formula II, and/or wherein the container containing a compound of Formula IA or Formula II does not contain docetaxel.

5. The kit according to claim 3 or 4, wherein the container containing docetaxel and the container containing a compound of Formula IA or Formula II are amongst, or represent, the at least two containers differing from each other in respect of their content in whole or in part.

6. The kit according to any one of claims 3 to 5, further comprising instructions to sequentially administer, to an individual suffering from a cancer or a tumor, a first pharmaceutical composition containing docetaxel and a second pharmaceutical composition containing a compound having the structure of Formula IA or Formula II within about 14 days of each other.

7. The pharmaceutical composition, use or kit of any one of claims 1 to 6, wherein said administration results in the sequential contact of a cell included in, derived from or being part of said cancer or tumor with docetaxel and a compound of Formula IA or Formula II within about 14 days of each other.

8. The pharmaceutical composition, use or kit of any one of claims 1 to 6, wherein said administration:
(i) is the sequential administration to said individual of first docetaxel and then a compound of Formula IA or Formula II within about 14 days of each other;
(ii) results in the sequential contact of a cell included in, derived from or being part of said cancer or tumor with first docetaxel and then a compound of Formula IA or Formula II within about 14 days of each other;
(iii) is the sequential administration to said individual of first a compound of Formula IA or Formula II and then docetaxel within about 14 days of each other; or
(iv) results in the sequential contact of a cell included in, derived from or being part of said cancer or tumor with first a compound of Formula IA or Formula II and then docetaxel within about 14 days of each other.

9. The pharmaceutical composition, use or kit of any one of claims 1 to 8, wherein said administration:
(i) is the sequential administration to said individual of docetaxel and a compound of Formula IA or Formula II within about 10 days, 7 days, 5 days, 3 days, 2 days or 1 day of each other; or
(ii) results in the sequential contact of a cell included in, derived from or being part of said cancer or tumour with docetaxel and a compound of Formula IA or Formula II within about 10 days, 7 days, 5 days, 3 days, 2 days or 1 day of each other.

10. The pharmaceutical composition, use or kit of any one of claims 1 to 9, wherein said administration:
(i) is the sequential administration to said individual of docetaxel and a compound of Formula IA or Formula II within about 48 hours, 24 hours, 12 hours, 8 hours, 6 hours, 4 hours, 2 hours, 1 hour, 30 mins, 15 mins or 5 mins of each other; or
(ii) results in the sequential contact of a cell included in, derived from or being part of said cancer or tumour with docetaxel and a compound of Formula IA or Formula II within about 48 hours, 24 hours, 12 hours, 8 hours, 6 hours, 4 hours, 2 hours, 1 hour, 30 mins, 15 mins or 5 mins of each other.

11. The pharmaceutical composition, use or kit of any one of claims 1 to 10, wherein said cancer or said tumor is included in, derived from or is a solid tumor.

12. The pharmaceutical composition, use or kit of claim 11, wherein said solid tumor is selected from: breast cancer, cervical cancer, colorectal cancer, peritoneal cancer, ovarian cancer, bronchial cancer, small cell lung cancer, non-small cell lung cancer, gastric, and head and neck cancer, or metastases thereof.

13. The pharmaceutical composition, use or kit of claim 11, wherein said solid tumor is prostate cancer.

14. The pharmaceutical composition, use or kit of any one of claims 1 to 10, wherein said cancer or said tumor is included in, derived from or is a hematological tumor.

15. The pharmaceutical composition, use or kit of claim 13, wherein said prostate cancer is hormone-refractory prostate cancer.

16. The pharmaceutical composition, use or kit of any one of claims 1 to 14, wherein said cancer or said tumor is a tumor or a cancer resistant or refractory to an anti-proliferative agent.

17. The pharmaceutical composition, use or kit of claim 16, wherein said anti-proliferative agent is cisplatin.

18. The pharmaceutical composition, use or kit of claim 16, wherein said anti-proliferative agent is not a hormone-based drug.

19. The pharmaceutical composition, use or kit of any one of claims 1 to 18, wherein the pharmaceutical composition containing a compound having the structure of Formula IA or Formula II is administered orally.

20. The pharmaceutical composition, use or kit of any one of claims 1 to 19, wherein the pharmaceutical composition containing docetaxel is administered intravenously.

21. The pharmaceutical composition, use or kit of any one of claims 1 to 20, wherein the compound of Formula IA or Formula II is administered at a dose of about 1 microgram per kilogram to about 500 milligrams per kilogram, about 100 micrograms per kilogram to about 50 milligrams per kilogram, or about 1 milligram per kilogram to about 5 milligrams per kilogram subject or sample weight.

22. The pharmaceutical composition, use or kit of any one of claims 1 to 20, wherein the compound of Formula IA or Formula II is administered at a dose of about 1 milligram per kilogram to about 5 milligrams per kilogram subject or sample weight.

23. The pharmaceutical composition, use or kit of any one of claims 1 to 20, wherein the compound of Formula IA or Formula II is administered at a dose of about 50 microgram per square meter to about 15 grams per square meter, about 5 milligrams per square meter to about 1.5 grams per square meter, or about 50 milligrams per square meter to about 150 milligrams per square meter body surface area.

24. The pharmaceutical composition, use or kit of any one of claims 1 to 20, wherein the compound of Formula IA or Formula II is administered at a dose of about 50 milligrams per square meter to about 150 milligrams per square meter body surface area.

25. The pharmaceutical composition, use or kit of any one of claims to 24, wherein docetaxel is administered at a dose of about 1 microgram per kilogram to about 500 milligrams per kilogram, about 100 micrograms per kilogram to about 50 milligrams per kilogram, or about 1 milligram per kilogram to about 5 milligrams per kilogram subject or sample weight.

26. The pharmaceutical composition, use or kit of any one of claims 1 to 24, wherein docetaxel is administered at a dose of about 1 milligram per kilogram to about 5 milligrams per kilogram subject or sample weight.

27. The pharmaceutical composition, use or kit of any one of claims 1 to 24, wherein docetaxel is administered at a dose of about 50 microgram per square meter to about 15 grams per square meter, about 5 milligrams per square meter to about 1.5 grams per square meter, or about 50 milligrams per square meter to about 150 milligrams per square meter body surface area.

28. The pharmaceutical composition, use or kit of any one of claims 1 to 24, wherein docetaxel is administered at a dose of about 50 milligrams per square meter to about 150 milligrams per square meter body surface area.

29. The pharmaceutical composition, use or kit of any one of claims 1 to 28, wherein said compound is the compound of Formula IA.

30. The pharmaceutical composition, use or kit of any one of claims 1 to 29, wherein said administration comprises co-administration with another drug.

31. The pharmaceutical composition, use or kit of claim 30, wherein said other drug is a chemotherapeutic agent, including anti-cancer therapeutic agents that inhibit cancer growth, anti-angiogenesis agents and anti-metastatic agents.

32. The pharmaceutical composition, use or kit of claim 30, wherein said other drug is an immunomodulator.

33. The pharmaceutical composition, use or kit of claim 30, wherein said administration is to a patient to whom an anti-emetic agent is also administered, including an anti-emetic agent selected from: granisetron, ondansetron, and tropisetron.

34. The pharmaceutical composition, use or kit of claim 30, wherein said administration is to a patient who is also treated with an anti-diarrheal, a corticosteroid, a growth hormone or growth factor, a diuretic, a steroidal or non-steroidal analgesic, or an anti-hyperuricemic.

35. The pharmaceutical composition, use or kit of claim 34, wherein said patient is also treated with GCSF or erythropoietin.

36. The pharmaceutical composition, use or kit of claim 34, wherein said patient is also treated with an opiate or paracetamol.

37. The pharmaceutical composition, use or kit of claims 24 or 28, wherein said individual is a person.

## Patentansprüche

1. Eine erste pharmazeutische Zusammensetzung, enthaltend entweder (i) eine Verbindung mit der folgenden Struktur: 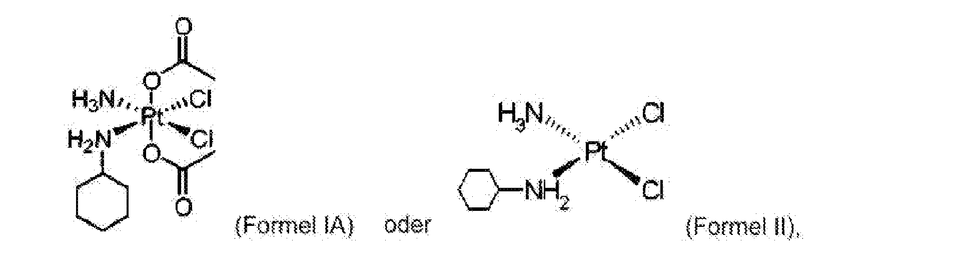 oder (ii) Docetaxel, wobei diese erste pharmazeutische Zusammensetzung für die Verwendung bei der Behandlung eines an einem Krebs oder einem Tumor leidenden Individuums ist, indem aufeinander folgend diese erste pharmazeutische Zusammensetzung und eine zweite pharmazeutische Zusammensetzung, die im Falle von (i) Docetaxel oder im Falle von (ii) eine Verbindung wie in (i) beschrieben enthält, innerhalb von etwa 14 Tagen voneinander verabreicht werden.

2. Verwendung von entweder (i) einer Verbindung der Formel IA oder Formel II oder (ii) Docetaxel, zur Herstellung einer ersten pharmazeutischen Zusammensetzung zur Behandlung eines an einem Krebs oder einem Tumor leidenden Individuums, wobei diese erste pharmazeutische Zusammensetzung innerhalb von etwa 14 Tagen auf die Verabreichung einer zweiten pharmazeutischen, im Falle von (i) Docetaxel oder im Falle von (ii) eine Verbindung der Formel IA oder Formel II enthaltenden Zusammensetzung folgend verabreicht werden soll.

3. Kit zur Verabreichung einer ersten und einer zweiten pharmazeutischen Zusammensetzung an ein an einem Krebs oder einem Tumor leidendes Individuum, wobei dieses Kit eine Vielzahl von getrennten Behältern aufweist, wobei sich der Inhalt von mindestens zwei dieser Behälter voneinander vollkommen oder teilweise unterscheidet, wobei mindestens einer dieser Behälter Docetaxel, mit oder ohne zusätzlichen pharmazeutischen Träger oder Verdünnungsmittel, enthält und mindestens ein anderer Behälter eine Verbindung der Formel IA oder Formel II, mit oder ohne zusätzlichen pharmazeutischen Träger oder Verdünnungsmittel, enthält.

4. Kit nach Anspruch 3, wobei der Docetaxel enthaltende Behälter keine Verbindung der Formel IA oder Formel II enthält und/oder wobei der eine Verbindung der Formel IA oder Formel II enthaltende Behälter kein Docetaxel enthält.

5. Kit nach Anspruch 3 oder 4, wobei der Docetaxel enthaltende Behälter und der eine Verbindung der Formel IA oder Formel II enthaltende Behälter sich unter den mindestens zwei Behältern befinden oder diese darstellen, die sich in Bezug auf ihren Inhalt vollständig oder teilweise voneinander unterscheiden.

6. Kit nach einem der Ansprüche 3 bis 5, des Weiteren umfassend Anweisungen zum aufeinander folgenden Verabreichen an ein an einem Krebs oder einem Tumor leidendes Individuum einer ersten, Docetaxel enthaltenden pharmazeutischen Zusammensetzung und einer zweiten, eine Verbindung mit der Struktur der Formel IA oder Formel II enthaltenden pharmazeutischen Zusammensetzung innerhalb von etwa 14 Tagen voneinander.

7. Pharmazeutische Zusammensetzung, Verwendung oder Kit nach einem der Ansprüche 1 bis 6, wobei die Verabreichung zu einem aufeinander folgenden Kontakt einer Zelle, die sich in diesem Krebs oder Tumor befindet, davon abgeleitet ist oder ein Teil davon ist, mit Docetaxel und einer Verbindung der Formel IA oder Formel II innerhalb von etwa 14 Tagen voneinander führt.

8. Pharmazeutische Zusammensetzung, Verwendung oder Kit nach einem der Ansprüche 1 bis 6, wobei die Verabreichung:
(i) eine aufeinander folgende Verabreichung an dieses Individuum von zunächst Docetaxel und dann einer Verbindung der Formel IA oder Formel II innerhalb von etwa 14 Tagen voneinander ist;
(ii) zu einem aufeinander folgenden Kontakt einer Zelle, die sich in diesem Krebs oder Tumor befindet, davon abgeleitet ist oder ein Teil davon ist, zunächst mit Docetaxel und dann mit einer Verbindung der Formel IA oder Formel II innerhalb von etwa 14 Tagen voneinander führt;
(iii) eine aufeinander folgende Verabreichung an dieses Individuum von zunächst einer Verbindung der Formel IA oder Formel II und dann Docetaxel innerhalb von etwa 14 Tagen voneinander ist; oder
(iv) zu einem aufeinander folgenden Kontakt einer Zelle, die sich in diesem Krebs oder Tumor befindet, davon abgeleitet ist oder ein Teil davon ist, zunächst mit einer Verbindung der Formel IA oder Formel II und dann mit Docetaxel innerhalb von etwa 14 Tagen voneinander führt.

9. Pharmazeutische Zusammensetzung, Verwendung oder Kit nach einem der Ansprüche 1 bis 8, wobei die Verabreichung:
(i) eine aufeinander folgende Verabreichung an dieses Individuum von Docetaxel und einer Verbindung der Formel IA oder Formel II innerhalb von etwa 10 Tagen, 7 Tagen, 5 Tagen, 3 Tagen, 2 Tagen oder 1 Tag voneinander ist; oder
(ii) zu einem aufeinander folgenden Kontakt einer Zelle, die sich in diesem Krebs oder Tumor befindet, davon abgeleitet ist oder ein Teil davon ist, mit Docetaxel und einer Verbindung der Formel IA oder Formel II innerhalb von etwa 10 Tagen, 7 Tagen, 5 Tagen, 3 Tagen, 2 Tagen oder 1 Tag voneinander führt.

10. Pharmazeutische Zusammensetzung, Verwendung oder Kit nach einem der Ansprüche 1 bis 9, wobei die Verabreichung:
(i) die aufeinander folgende Verabreichung an dieses Individuum von Docetaxel und einer Verbindung der Formel IA oder Formel II innerhalb von etwa 48 Stunden, 24 Stunden, 12 Stunden, 8 Stunden, 6 Stunden, 4 Stunden, 2 Stunden, 1 Stunde, 30 min, 15 min oder 5 min voneinander ist; oder
(ii) zu einem aufeinander folgenden Kontakt einer Zelle, die sich in diesem Krebs oder Tumor befindet, davon abgeleitet ist oder ein Teil davon ist, mit Docetaxel und einer Verbindung der Formel IA oder Formel II innerhalb von etwa 48 Stunden, 24 Stunden, 12 Stunden, 8 Stunden, 6 Stunden, 4 Stunden, 2 Stunden, 1 Stunde, 30 min, 15 min oder 5 min voneinander führt.

11. Pharmazeutische Zusammensetzung, Verwendung oder Kit nach einem der Ansprüche 1 bis 10, wobei sich dieser Krebs oder dieser Tumor in einem soliden Tumor befindet, davon abgeleitet ist oder ein solider Tumor ist.

12. Pharmazeutische Zusammensetzung, Verwendung oder Kit nach Anspruch 11, wobei dieser solide Tumor ausgewählt wird aus: Brustkrebs, Gebärmutterhalskrebs, Kolorektalkrebs, Peritonealkrebs, Eierstockkrebs, Bronchialkrebs, kleinzelliger Lungenkrebs, nichtkleinzelliger Lungenkrebs, Magenkrebs und Kopf- und Halskrebs oder Metastasen davon.

13. Pharmazeutische Zusammensetzung, Verwendung oder Kit nach Anspruch 11, wobei dieser solide Tumor Prostatakrebs ist.

14. Pharmazeutische Zusammensetzung, Verwendung oder Kit nach einem der Ansprüche 1 bis 10, wobei sich dieser Krebs oder dieser Tumor in einem hämatologischen Tumor befindet, davon abgeleitet ist oder ein hämatologischer Tumor ist.

15. Pharmazeutische Zusammensetzung, Verwendung oder Kit nach Anspruch 13, wobei dieser Prostatakrebs hormon-refraktärer Prostatakrebs ist.

16. Pharmazeutische Zusammensetzung, Verwendung oder Kit nach einem der Ansprüche 1 bis 14, wobei dieser Krebs oder dieser Tumor ein gegenüber einem antiproliferativen Mittel resistenter oder refraktärer Tumor oder Krebs ist.

17. Pharmazeutische Zusammensetzung, Verwendung oder Kit nach Anspruch 16, wobei dieses antiproliferative Mittel Cisplatin ist.

18. Pharmazeutische Zusammensetzung, Verwendung oder Kit nach Anspruch 16, wobei dieses antiproliferative Mittel kein Wirkstoff auf Hormonbasis ist.

19. Pharmazeutische Zusammensetzung, Verwendung oder Kit nach einem der Ansprüche 1 bis 18, wobei die eine Verbindung mit der Struktur der Formel IA oder Formel II enthaltende pharmazeutische Zusammensetzung oral verabreicht wird.

20. Pharmazeutische Zusammensetzung, Verwendung oder Kit nach einem der Ansprüche 1 bis 19, wobei die Docetaxel enthaltende pharmazeutische Zusammensetzung intravenös verabreicht wird.

21. Pharmazeutische Zusammensetzung, Verwendung oder Kit nach einem der Ansprüche 1 bis 20, wobei die Verbindung der Formel IA oder Formel II mit einer Dosis von etwa 1 Mikrogramm pro Kilogramm bis etwa 500 Milligramm pro Kilogramm, etwa 100 Mikrogramm pro Kilogramm bis etwa 50 Milligramm pro Kilogramm oder etwa 1 Milligramm pro Kilogramm bis etwa 5 Milligramm pro Kilogramm Gewicht des Subjekts oder der Probe verabreicht wird.

22. Pharmazeutische Zusammensetzung, Verwendung oder Kit nach einem der Ansprüche 1 bis 20, wobei die Verbindung der Formel IA oder Formel II mit einer Dosis von etwa 1 Milligramm pro Kilogramm bis etwa 5 Milligramm pro Kilogramm Gewicht des Subjekts oder der Probe verabreicht wird.

23. Pharmazeutische Zusammensetzung, Verwendung oder Kit nach einem der Ansprüche 1 bis 20, wobei die Verbindung der Formel IA oder Formel II mit einer Dosis von etwa 50 Mikrogramm pro Quadratmeter bis etwa 15 Gramm pro Quadratmeter, etwa 5 Milligramm pro Quadratmeter bis etwa 1,5 Gramm pro Quadratmeter oder etwa 50 Milligramm pro Quadratmeter bis etwa 150 Milligramm pro Quadratmeter Körperoberfläche verabreicht wird.

24. Pharmazeutische Zusammensetzung, Verwendung oder Kit nach einem der Ansprüche 1 bis 20, wobei die Verbindung der Formel IA oder Formel II mit einer Dosis von etwa 50 Milligramm pro Quadratmeter bis etwa 150 Milligramm pro Quadratmeter Körperoberfläche verabreicht wird.

25. Pharmazeutische Zusammensetzung, Verwendung oder Kit nach einem der Ansprüche 1 bis 24, wobei Docetaxel mit einer Dosis von etwa 1 Mikrogramm pro Kilogramm bis etwa 500 Milligramm pro Kilogramm, etwa 100 Mikrogramm pro Kilogramm bis etwa 50 Milligramm pro Kilogramm oder etwa 1 Milligramm pro Kilogramm bis etwa 5 Milligramm pro Kilogramm Gewicht des Subjekts oder der Probe verabreicht wird.

26. Pharmazeutische Zusammensetzung, Verwendung oder Kit nach einem der Ansprüche 1 bis 24, wobei Docetaxel mit einer Dosis von etwa 1 Milligramm pro Kilogramm bis etwa 5 Milligramm pro Kilogramm Gewicht des Subjekts oder der Probe verabreicht wird.

27. Pharmazeutische Zusammensetzung, Verwendung oder Kit nach einem der Ansprüche 1 bis 24, wobei Docetaxel mit einer Dosis von etwa 50 Mikrogramm pro Quadratmeter bis etwa 15 Gramm pro Quadratmeter, etwa 5 Milligramm pro Quadratmeter bis etwa 1,5 Gramm pro Quadratmeter oder etwa 50 Milligramm pro Quadratmeter bis etwa 150 Milligramm pro Quadratmeter Körperoberfläche verabreicht wird.

28. Pharmazeutische Zusammensetzung, Verwendung oder Kit nach einem der Ansprüche 1 bis 24, wobei Docetaxel mit einer Dosis von etwa 50 Milligramm pro Quadratmeter bis etwa 150 Milligramm pro Quadratmeter Körperoberfläche verabreicht wird.

29. Pharmazeutische Zusammensetzung, Verwendung oder Kit nach einem der Ansprüche 1 bis 28, wobei die Verbindung eine Verbindung der Formel IA ist.

30. Pharmazeutische Zusammensetzung, Verwendung oder Kit nach einem der Ansprüche 1 bis 29, wobei die Verabreichung eine gemeinsame Verabreichung mit einem weiteren Wirkstoff umfasst.

31. Pharmazeutische Zusammensetzung, Verwendung oder Kit nach Anspruch 30, wobei dieser weitere Wirkstoff ein chemotherapeutisches Mittel ist, einschließlich therapeutischer Antikrebsmittel, die das Wachstum von Krebs inhibieren, Antiangiogenesemittel und antimetastatische Mittel.

32. Pharmazeutische Zusammensetzung, Verwendung oder Kit nach Anspruch 30, wobei dieser weitere Wirkstoff ein Immunmodulator ist.

33. Pharmazeutische Zusammensetzung, Verwendung oder Kit nach Anspruch 30, wobei die Verabreichung an einen Patienten erfolgt, dem auch ein antiemetisches Mittel verabreicht wird, einschließlich ein antiemetisches Mittel ausgewählt aus: Granisetron, Ondansetron und Tropisetron.

34. Pharmazeutische Zusammensetzung, Verwendung oder Kit nach Anspruch 30, wobei die Verabreichung an einen Patienten erfolgt, der auch mit einem Antidiarrhoikum, einem Kortikosteroid, einem Wachstumshormon oder einem Wachstumsfaktor, einem Diuretikum, einem steroidalen oder nichtsteroidalen Analgetikum oder einem antihyperurikämisch wirkenden Mittel behandelt wird.

35. Pharmazeutische Zusammensetzung, Verwendung oder Kit nach Anspruch 34, wobei dieser Patient auch mit GCSF oder Erythropoetin behandelt wird.

36. Pharmazeutische Zusammensetzung, Verwendung oder Kit nach Anspruch 34, wobei dieser Patient auch mit einem Opiat oder Paracetamol behandelt wird.

37. Pharmazeutische Zusammensetzung, Verwendung oder Kit nach einem der Ansprüche 24 oder 28, wobei dieses Individuum eine Person ist.

## Revendications

1. Première composition pharmaceutique contenant soit (i) un composé présentant la structure de : 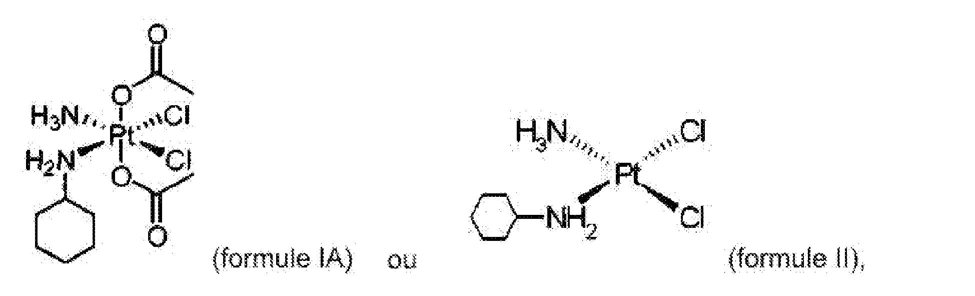 ou (ii) du docetaxel, dans lequel ladite première composition pharmaceutique est destinée à être utilisée dans le traitement d'un individu souffrant d'un cancer ou d'une tumeur par administration séquentielle de ladite première composition pharmaceutique et d'une deuxième composition pharmaceutique contenant, dans le cas (i), du docetaxel, ou dans le cas (ii), un composé comme celui décrit à (i) en l'espace de 14 jours l'une après l'autre.

2. Utilisation de soit (i) un composé selon la Formule IA ou de la Formule II, ou (ii) de docetaxel pour la préparation d'une première composition pharmaceutique pour le traitement d'un individu souffrant d'un cancer ou d'une tumeur, étant donné que ladite première composition pharmaceutique doit être administrée séquentiellement en l'espace d'environ 14 jours après l'administration d'une deuxième composition pharmaceutique contenant, dans le cas (i), du docetaxel, ou dans le cas (ii), un composé selon la Formule IA ou de la Formule II.

3. Kit pour l'administration d'une première et d'une deuxième composition pharmaceutiques à un individu souffrant d'un cancer ou d'une tumeur, étant donné que ledit kit contient une pluralité de conteneurs séparés, les contenus d'au moins deux conteneurs différant totalement ou partiellement l'un de l'autre, étant donné qu'au moins un de ces conteneurs contient du docetaxel, avec ou sans porteur ou diluant pharmaceutique additionnel, et au moins un conteneur différent contient un composé selon la Formule IA ou la Formule II, avec ou sans porteur ou diluant pharmaceutique additionnel.

4. Kit selon la revendication 3, dans lequel le conteneur contenant du docetaxel ne contient pas un composé selon la Formule IA ou la Formule II, et/ou dans lequel le conteneur contenant un composé selon la Formule IA ou la Formule II ne contient pas du docetaxel.

5. Kit selon la revendication 3 ou 4, dans lequel le conteneur contenant du docetaxel et le conteneur contenant un composé selon la Formule IA ou la Formule II font partie des ou représentent les au moins deux conteneurs dont les contenus diffèrent totalement ou partiellement l'un de l'autre.

6. Kit selon l'une quelconque des revendications 3 à 5, comprenant en outre des instructions pour administrer séquentiellement à un individu souffrant d'un cancer ou d'une tumeur une première composition pharmaceutique contenant du docetaxel et une deuxième composition pharmaceutique contenant un composé présentant la structure de la Formule IA ou la Formule II en l'espace d'environ 14 jours l'une après l'autre.

7. Composition pharmaceutique, utilisation ou kit selon l'une quelconque des revendications 1 à 6, où ladite administration entraîne le contact séquentiel d'une cellule incluse dans, dérivée du ou faisant partie dudit cancer ou de ladite tumeur avec du docetaxel et un composé selon la Formule IA ou la Formule II en l'espace d'environ 14 jours l'un après l'autre.

8. Composition pharmaceutique, utilisation ou kit selon l'une quelconque des revendications 1 à 6, où ladite administration :
(i) est l'administration séquentielle audit individu tout d'abord de docetaxel, puis d'un composé selon la Formule IA ou la Formule II en l'espace d'environ 14 jours l'une après l'autre ;
(ii) entraîne le contact séquentiel d'une cellule incluse dans, dérivée du ou faisant partie dudit cancer ou de ladite tumeur avec tout d'abord du docetaxel, puis un composé selon la Formule IA ou la Formule II en l'espace d'environ 14 jours l'un après l'autre ;
(iii) est l'administration séquentielle audit individu tout d'abord d'un composé selon la Formule IA ou la Formule II, puis de docetaxel en l'espace d'environ 14 jours l'une après l'autre ;
(iv) entraîne le contact séquentiel d'une cellule incluse dans, dérivée du ou faisant partie dudit cancer ou de ladite tumeur avec tout d'abord un composé selon la Formule IA ou la Formule II, puis du docetaxel en l'espace d'environ 14 jours l'un après l'autre ;

9. Composition pharmaceutique, utilisation ou kit selon l'une quelconque des revendications 1 à 8, où ladite administration :
(i) est l'administration séquentielle audit individu de docetaxel et d'un composé selon la Formule IA ou la Formule II en l'espace d'environ 10 jours, 7 jours, 5 jours, 3 jours, 2 jours ou 1 jour l'une après l'autre ;
(ii) entraîne le contact séquentiel d'une cellule incluse dans, dérivée du ou faisant partie dudit cancer ou de ladite tumeur avec du docetaxel et un composé selon la Formule IA ou la Formule II en l'espace d'environ 10 jours, 7 jours, 5 jours, 3 jours, 2 jours ou 1 jour l'un après l'autre.

10. Composition pharmaceutique, utilisation ou kit selon l'une quelconque des revendications 1 à 9, où ladite administration :
(i) est l'administration séquentielle audit individu de docetaxel et d'un composé selon la Formule IA ou la Formule II en l'espace d'environ 48 heures, 24 heures, 12 heures, 8 heures, 6 heures, 4 heures, 2 heures, 1 heure, 30 mn, 15 mn ou 5 mn l'une après l'autre ; ou
(ii) entraîne le contact séquentiel d'une cellule incluse dans, dérivée du ou faisant partie dudit cancer ou de ladite tumeur avec du docetaxel et un composé selon la Formule IA ou la Formule II en l'espace d'environ 48 heures, 24 heures, 12 heures, 8 heures, 6 heures, 4 heures, 2 heures, 1 heure, 30 mn, 15 mn ou 5 mn l'un après l'autre.

11. Composition pharmaceutique, utilisation ou kit selon l'une quelconque des revendications 1 à 10, où ledit cancer ou ladite tumeur est inclus(e) dans, dérivé(e) de ou est une tumeur solide.

12. Composition pharmaceutique, utilisation ou kit selon la revendication 11, où ladite tumeur solide est sélectionnée dans le groupe se composant de : cancer du sein, cancer du cerveau, cancer colorectal, cancer du péritoine, cancer ovarien, cancer bronchique, cancer des poumons à petites cellules, cancer des poumons non à petites cellules, cancer gastrique et cancer de la tête et du cou, ou des métastases de ces derniers.

13. Composition pharmaceutique, utilisation ou kit selon la revendication 11, où ladite tumeur solide est un cancer de la prostate.

14. Composition pharmaceutique, utilisation ou kit selon l'une quelconque des revendications 1 à 10, où ledit cancer ou ladite tumeur est inclus(e) dans, dérivé(e) de ou est une tumeur hématologique.

15. Composition pharmaceutique, utilisation ou kit selon la revendication 13, où ledit cancer de la prostate est un cancer de la prostate réfractaire aux hormones.

16. Composition pharmaceutique, utilisation ou kit selon l'une quelconque des revendications 1 à 14, où ledit cancer ou ladite tumeur est une tumeur ou un cancer qui est résistant(e) ou réfractaire à un agent anti-prolifératif.

17. Composition pharmaceutique, utilisation ou kit selon la revendication 16, où ledit agent anti-prolifératif est le cisplatin.

18. Composition pharmaceutique, utilisation ou kit selon la revendication 16, où ledit agent anti-prolifératif n'est pas un médicament à base d'hormone.

19. Composition pharmaceutique, utilisation ou kit selon l'une quelconque des revendications 1 à 18, où la composition pharmaceutique contenant un composé présentant la structure de la Formule IA ou la Formule II est administrée oralement.

20. Composition pharmaceutique, utilisation ou kit selon l'une quelconque des revendications 1 à 19, où la composition pharmaceutique contenant du docetaxel est administrée intraveineusement.

21. Composition pharmaceutique, utilisation ou kit selon l'une quelconque des revendications 1 à 20, où le composé selon la Formule IA ou la Formule II est administré à une dose d'environ 1 microgramme par kilogramme à environ 500 milligrammes par kilogramme, environ 100 microgrammes par kilogramme à environ 50 milligrammes par kilogramme, ou environ 1 milligramme par kilogramme à environ 5 milligrammes par kilogramme de poids du sujet ou d'échantillon.

22. Composition pharmaceutique, utilisation ou kit selon l'une quelconque des revendications 1 à 20, où le composé selon la Formule IA ou la Formule II est administré à une dose d'environ 1 milligramme par kilogramme à environ 5 milligrammes par kilogramme de poids du sujet ou d'échantillon.

23. Composition pharmaceutique, utilisation ou kit selon l'une quelconque des revendications 1 à 20, où le composé selon la Formule IA ou la Formule II est administré à une dose d'environ 50 microgrammes par mètre carré à environ 15 grammes par mètre carré, environ 5 milligrammes par mètre carré à environ 1,5 grammes par mètre carré, ou environ 50 milligrammes par mètre carré à environ 150 milligrammes par mètre carré de surface du corps.

24. Composition pharmaceutique, utilisation ou kit selon l'une quelconque des revendications 1 à 20, où le composé selon la Formule IA ou la Formule II est administré à une dose d'environ 50 milligrammes par mètre carré à environ 150 milligrammes par mètre carré de surface du corps.

25. Composition pharmaceutique, utilisation ou kit selon l'une quelconque des revendications 1 à 24, où le docetaxel est administré à une dose d'environ 1 microgramme par kilogramme à environ 500 milligrammes par kilogramme, environ 100 microgrammes par kilogramme à environ 50 milligrammes par kilogramme, ou environ 1 milligramme par kilogramme à environ 5 milligrammes par kilogramme de poids du sujet ou d'échantillon.

26. Composition pharmaceutique, utilisation ou kit selon l'une quelconque des revendications 1 à 24, où le docetaxel est administré à une dose d'environ 1 milligramme par kilogramme à environ 5 milligrammes par kilogramme de poids du sujet ou d'échantillon.

27. Composition pharmaceutique, utilisation ou kit selon l'une quelconque des revendications 1 à 24, où le docetaxel est administré à une dose d'environ 50 microgrammes par mètre carré à environ 15 grammes par mètre carré, environ 5 milligrammes par mètre carré à environ 1,5 grammes par mètre carré, ou environ 50 milligrammes par mètre carré à environ 150 milligrammes par mètre carré de surface du corps.

28. Composition pharmaceutique, utilisation ou kit selon l'une quelconque des revendications 1 à 24, où le docetaxel est administré à une dose d'environ 50 milligramme par mètre carré à environ 150 milligrammes par mètre carré de surface du corps.

29. Composition pharmaceutique, utilisation ou kit selon l'une quelconque des revendications 1 à 28, où ledit composé est le composé selon la Formule IA.

30. Composition pharmaceutique, utilisation ou kit selon l'une quelconque des revendications 1 à 29, où ladite administration comprend la co-administration avec un autre médicament.

31. Composition pharmaceutique, utilisation ou kit selon la revendication 30, où ledit autre médicament est un agent chimiothérapeutique, y compris des agents thérapeutiques anti-cancer qui inhibent la croissance du cancer, des agents anti-angiogénétiques et agents anti-métastatiques.

32. Composition pharmaceutique, utilisation ou kit selon la revendication 30, où ledit autre médicament est un immunomodulateur.

33. Composition pharmaceutique, utilisation ou kit selon la revendication 30, où ladite administration est faite à un patient à qui un agent antiémétique est également administré, y compris un agent antiémétique sélectionné dans le groupe se composant de : granisétron, ondansétron et tropisétron.

34. Composition pharmaceutique, utilisation ou kit selon la revendication 30, où ladite administration est faite à un patient qui est également traité avec un agent anti-diarrhée, un corticostéroïde, une hormone de croissance ou un facteur de croissance, un diurétique, un analgésique stéroïdique ou non-stéroïdique, ou un anti-hyperuricémique.

35. Composition pharmaceutique, utilisation ou kit selon la revendication 34, où ledit patient est également traité avec du GCSF ou de l'érythropoïétine.

36. Composition pharmaceutique, utilisation ou kit selon la revendication 34, où ledit patient est également traité avec un opiat ou du paracétamol.

37. Composition pharmaceutique, utilisation ou kit selon les revendications 24 ou 28, où ledit individu est une personne.
